(19) Europäisches Patentamt / European Patent Office / Office européen des brevets

(11) EP 1 808 433 A1

(12) **EUROPEAN PATENT APPLICATION**
published in accordance with Art. 158(3) EPC

(43) Date of publication:
**18.07.2007 Bulletin 2007/29**

(21) Application number: **05795391.1**

(22) Date of filing: **19.10.2005**

(51) Int Cl.:
***C07D 403/04*** *(2006.01)*

(86) International application number:
**PCT/JP2005/019233**

(87) International publication number:
**WO 2006/049013 (11.05.2006 Gazette 2006/19)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **04.11.2004 JP 2004320439**

(71) Applicant: **IDEMITSU KOSAN COMPANY LIMITED**
**Tokyo 100-0005 (JP)**

(72) Inventors:
- **IKEDA, Kiyoshi**
  **2990293 (JP)**
- **ITO, Mitsunori**
  **2990293 (JP)**
- **ARAKANE, Takashi**
  **2990293 (JP)**

(74) Representative: **Gille Hrabal Struck Neidlein Prop Roos**
**Brucknerstrasse 20**
**40593 Düsseldorf (DE)**

# (54) COMPOUND CONTAINING FUSED RING AND ORGANIC ELECTROLUMINESCENT ELEMENT EMPLOYING THE SAME

(57) Provided is a condensed ring-containing compound having a condensed dicyclic group having a specific structure and carbazolyl groups and/or indolyl groups, and further provided is an organic electroluminescent device in which an organic thin film layer comprising a single layer or plural layers having at least a light emitting layer is interposed between a cathode and an anode, wherein at least one layer in the above organic thin film layer contains the condensed ring-containing compound described above in the form of a single component or a mixed component, whereby a current efficiency and a heat resistance are high, and a lifetime is very long.

EP 1 808 433 A1

## Description

BACKGROUND OF THE INVENTION

The present invention relates to a novel condensed ring-containing compound and an organic electroluminescent (EL) device using the same, specifically to an organic electroluminescent device which has a high current efficiency and a high heat resistance and which has a very long lifetime and a condensed ring-containing compound which materializes the same.

RELATED ART

Organic EL devices in which an organic light emitting layer is interposed between electrodes have so far been actively researched and developed because of reasons shown below.

(1) They are completely solid devices and therefore easy to handle and produce.
(2) Spontaneous emission is possible, and therefore light emitting members are not necessary.
(3) They are excellent in visibility and therefore suited to displays.
(4) They can readily be turned into full-color devices.

In general, an emission mechanism of the above organic EL devices makes use of a fluorescent emission phenomenon (luminescent phenomenon) which is energy conversion brought about when fluorescent molecules staying in a singlet excitation state (referred to as an S1 state) radioactively transit to a ground state in an organic luminescent medium. Further, fluorescent molecules staying in a triplet excitation state (referred to as a T1 state) are assumed as well, but radioactive transition to a ground state is forbidden transition, so that such fluorescent molecules transit gradually from the triplet excitation state to another state by non-radioactive transition. As a result thereof, heat energy is discharged instead of fluorescent emission.

In this regard, a singlet and a triplet mean multiplicity of energy determined by the number of combinations of a whole spin angular momentum of a fluorescent molecule with a whole orbit angular momentum thereof. That is, a singlet excitation state is defined by an energy state observed when one electron is allowed to transit from a ground state in which unpaired electrons are not present to a higher energy level while a spin state of the electron stays unchanged. Further, a triplet excitation state is defined by an energy state observed when one electron is allowed to transit to a higher energy level while a spin state of the electron stays reversed. It is a matter of course that emission from the triplet excitation state defined above can be observed at a very low temperature, for example, a liquefied temperature (-196°C) of liquid nitrogen. However, it is not a practical temperature condition, and in addition thereto, it is only a small emission amount.

On the other hand, a whole efficiency of emission in conventional organic EL devices relates to a recombination efficiency ($Ø_{rec}$) of a charge carrier injected (electron and hole) and a probability ($Ø_{rad}$) at which an exciton produced brings about radioactive transition, and therefore a whole efficiency ($Ø_{e1}$) of emission in an organic EL device shall be expressed by the following equation:

$$\phi_{e1} = \phi_{rec} \times 0.25 \phi_{rad}$$

In this connection, a coefficient 0.25 in $Ø_{rad}$ of the equation is based on the consideration that a production probability of a singlet exciton is 1/4. Accordingly, assuming that radioactive decay of recombination and an exciton is brought about at a probability coefficient of 1, a theoretical upper limit in an emission efficiency of an organic EL device shall be 25 %. As shown above, a triplet exciton can not substantially be used in conventional organic EL devices, and only a singlet exciton brings about radioactive transition. Accordingly, the problem that an upper limit value of the emission efficiency is low, has been involved therein. Accordingly, it is tried to bring about a fluorescent emission phenomenon by transferring energy from a triplet exciton produced to a phosphorescent dopant even on a room temperature condition making use of a triplet exciton (triplet excitation state) of an organic luminescent material (host material) (refer to, for example, a non-patent document 1). To be more specific, it is reported that an emission phenomenon is caused by constituting an organic EL device comprising an organic luminescent layer constituted from 4,4-N,N-dicarbazolylbiphenyl and an Ir complex as a phosphorescent dopant.

Under the above circumstance, researches of phosphorescent devices making use of a triplet exciton have been advanced in recent years. According to, for example, patent documents 1 and 2, it is disclosed that specific compounds having a pyrimidine ring and a quinazoline ring have a high efficiency as electron transporting materials and that use of them for an electron transporting layer or an luminescent layer provides organic electroluminescent devices which are

improved in an emission luminance and a luminous efficiency and which are elongated in a life. Also, a specific compound group in which a triazine ring is combined with a carbazolyl group is disclosed as a host compound for a blue color in a patent document 3. Further, a compound group having both of a benzimidazolyl group which is a nitrogen-containing condensed dicyclic group formed by condensation of a five-membered ring and a six-membered ring and a carbazolyl group is shown in a patent document 4, but the device performances are not shown therein. Also, compounds having both of a nitrogen-containing condensed dicyclic group formed by condensation of two six-membered rings and a carbazolyl group are not shown therein, and examples in which the above compounds are used as a phosphorescent host material are not disclosed.

Patent document 1: Japanese Patent Application Laid-Open No. 031004/2003
Patent document 2: Japanese Patent Application Laid-Open No. 045662/2003
Patent document 3: Japanese Patent Application Laid-Open No. 193952/2002
Patent document 4: Japanese Patent Application Laid-Open No. 319419/2002
Non-patent document 1: Jpn. J. Appl. Phys., 38 (1999) L1502

DISCLOSURE OF THE INVENTION

The present invention has been made in order to solve the problems described above, and an object thereof is to provide an organic EL device which has a high current efficiency and a high heat resistance and which has long lifetime and a condensed ring-containing compound which materializes the same.

Intensive researches repeated by the present inventors in order to achieve the object described above have resulted in finding that use of a condensed ring-containing compound having a condensed dicyclic group having a specific structure, particularly a condensed dicyclic group formed by condensation of two six-membered rings and carbazolyl groups and/or indolyl groups as a host material for an organic EL device provides an organic EL device which can effectively emit light by making use of a triplet exciton state and which has a practical lifetime and is excellent in current efficiency and heat resistance, and thus the present invention has been completed.

That is, the present invention provides a condensed ring-containing compound having a condensed dicyclic group represented by the following Formula (1) and/or (2) and carbazolyl groups and/or indolyl groups represented by at lest one selected from the following Formulas (3) to (8):

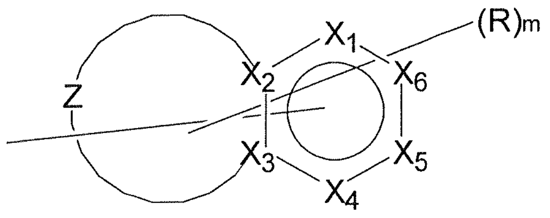

(1)

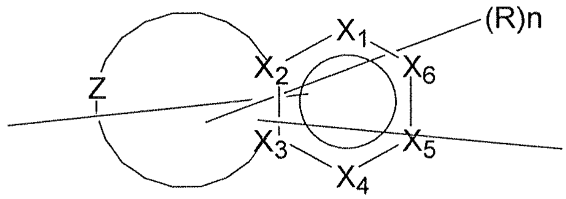

(2)

wherein $X_1$ to $X_6$ each are independently a nitrogen atom, an oxygen atom, a sulfur atom or a carbon atom; Z is an atomic group forming a cyclic structure; R is an aryl group having 6 to 50 ring carbon atoms which may have a substituent, a heterocyclic group having 5 to 50 ring atoms which may have a substituent, an alkyl group having 1 to 50 carbon atoms which may have a substituent, an alkoxy group having 1 to 50 carbon atoms which may have a substituent, an aralkyl group having 7 to 50 ring carbon atoms which may have a substituent, an aryloxy group having 5 to 50 ring carbon atoms which may have a substituent, an arylthio group having 5 to 50 ring carbon atoms which may have a substituent, an alkoxycarbonyl group having 1 to 50 carbon atoms which may have a substituent, a carboxyl group, a halogen atom, a cyano group, a nitro group or a hydroxyl group, and when R is plural, they may be combined with each other to form a cyclic structure; and

m and n each are an integer of 0 to 10;

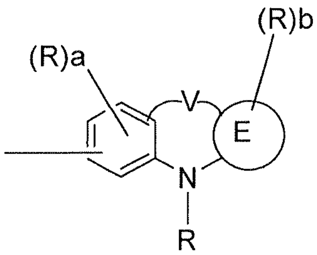
(3)
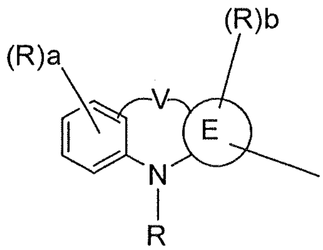
(4)
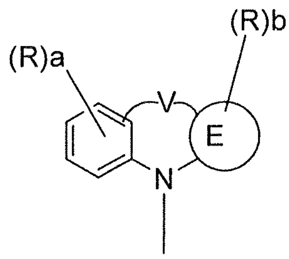
(5)

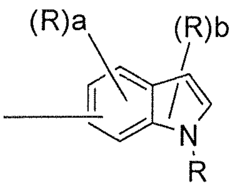
(6)
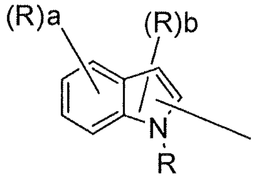
(7)
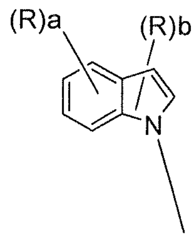
(8)

wherein R is the same as described above, and a and b each are an integer of 0 to 4;
V is a single bond, $-CR_0R_0'-$, $-SiR_0R_0'-$, -O-, -CO- or $-NR_0$ ($R_0$ and $R_0'$ each are independently a hydrogen atom, an aryl group having 6 to 50 ring carbon atoms which may have a substituent, a heterocyclic group having 5 to 50 ring atoms which may have a substituent or an alkyl group having 1 to 50 carbon atoms which may have a substituent);
E represents a cyclic structure shown by a circle surrounding the symbol E and is a cycloalkane residue having 3 to 20 ring carbon atoms which may have a substituent and in which a carbon atom may be replaced by a nitrogen atom, an aryl group having 4 to 50 ring carbon atoms which may have a substituent or a heterocyclic group having 4 to 50 ring atoms which may have a substituent.

Further, the present invention provides an organic EL device in which an organic thin film layer comprising a single layer or plural layers having at least a light emitting layer is interposed between a cathode and an anode, wherein at least one layer in the above organic thin film layer contains the condensed ring-containing compound described above in the form of a single component or a mixed component.

An organic EL device using the condensed ring-containing compound of the present invention is excellent in current efficiency and heat resistance and has a very long lifetime, and therefore it is practical.

BEST MODE FOR CARRYING OUT THE INVENTION

The condensed ring-containing compound of the present invention is a compound having a condensed dicyclic group represented by the following Formula (1) and/or (2) and carbazolyl groups and/or indolyl groups represented by at lest one selected from the following Formulas (3) to (8):

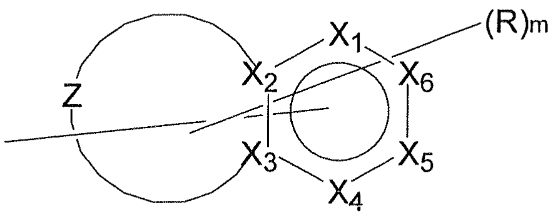
(1)
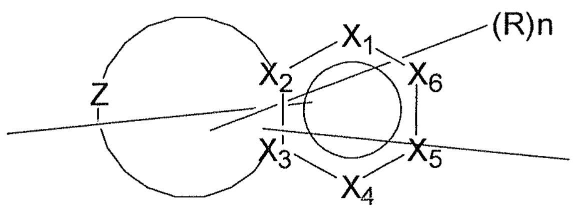
(2)

In Formulas (1) and (2), $X_1$ to $X_6$ each are independently a nitrogen atom, an oxygen atom, a sulfur atom or a carbon atom, and they are preferably a nitrogen atom.
Further, at least one of $X_1$, $X_2$ and $X_3$ is preferably a nitrogen atom.
In Formulas (1) and (2), Z is an atomic group forming a cyclic structure. The atomic group forming a cyclic structure includes, for example, alkylene groups such as an ethylene group, a propylene group, a n-butylene group, a n-pentylene group and a n-hexylene group and groups obtained by replacing at least one of carbon atoms in the above alkylene

groups by a nitrogen atom or an oxygen atom to form heterocycles, and they may have substituents. Further, the substituents may be combined with each other to form saturated or unsaturated cyclic structures. The specific examples of the cyclic structure include, for example, cycloalkanes having 4 to 12 carbon atoms such as cyclobutane, cyclopentane, cyclohexane, adamantane and norbornane, cycloalkenes having 4 to 12 carbon atoms such as cyclobutene, cyclopentene, cyclohexene, cycloheptene and cyclooctene, cycloalkadienes having 6 to 12 carbon atoms such as cyclohexadiene, cycloheptadiene and cyclooctadiene, aromatic rings having 6 to 50 carbon atoms such as benzene, naphthalene, phenanthrene, anthracene, pyrene, chrysene and acenaphthylene, heterocycles having 5 to 50 ring atoms such as pyrazole, imidazole, pyrazine, pyrimidine, indazole, purine, phthalazine, naphthylidine, quinoxaline, quinazoline, cinnoline, pteridine, perimidine, phenanthroline, pyrroloimidazole, pyrrolotriazole, pyrazoloimidazole, pyrazolotriazole, pyrazolopyrimidine, pyrazolotriazine, imidazoimidazole, imidazopyridazine, imidazopyridine, imidazopyrazine, triazolopyridine, benzimidazole, naphthoimidazole, benzoxazole, naphthoxazole, benzothiazole, naphthothiazole, benzotriazole, tetrazaindene, triazine and carbazole.

In Formulas (1) and (2), R is an aryl group having 6 to 50 ring carbon atoms which may have a substituent, a heterocyclic group having 5 to 50 ring atoms which may have a substituent, an alkyl group having 1 to 50 carbon atoms which may have a substituent, an alkoxy group having 1 to 50 carbon atoms which may have a substituent, an aralkyl group having 7 to 50 ring carbon atoms which may have a substituent, an aryloxy group having 5 to 50 ring carbon atoms which may have a substituent, an arylthio group having 5 to 50 ring carbon atoms which may have a substituent, an alkoxycarbonyl group having 1 to 50 carbon atoms which may have a substituent, a carboxyl group, a halogen atom, a cyano group, a nitro group or a hydroxyl group, and when R is plural, they may be combined with each other to form a cyclic structure.

The cyclic structures thereof include the same ones as explained in Z described above.

In Formula (1), m is an integer of 0 to 10, preferably 1 to 5, and in Formula (2), n is an integer of 0 to 10, preferably 1 to 5.

The examples of the aryl group represented by R described above include phenyl, 1-naphthyl, 2-naphthyl, 1-anthryl, 2-anthryl, 9-anthryl, 1-phenanthryl, 2-phenanthryl, 3-phenanthryl, 4-phenanthryl, 9-phenanthryl, 1-naphthacenyl, 2-naphthacenyl, 9-naphthacenyl, 1-pyrenyl, 2-pyrenyl, 4-pyrenyl, 2-biphenylyl, 3-biphenylyl, 4-biphenylyl, p-terphenyl-4-yl, p-terphenyl-3-yl, p-terphenyl-2-yl, m-terphenyl-4-yl, m-terphenyl-3-yl, m-terphenyl-2-yl, o-tolyl, m-tolyl, p-tolyl, p-t-butylphenyl, p-(2-phenylpropyl)phenyl, 3-methyl-2-naphthyl, 4-methyl-1-naphthyl, 4-methyl-l-anthryl, 4'-methylbiphenylyl and 4"-t-butyl-p-terphenyl-4-yl.

The examples of the heterocyclic group represented by R described above include 1-pyrrolyl, 2-pyrrolyl, 3-pyrrolyl, pyrazinyl, 2-pyridinyl, 3-pyridinyl, 4-pyridinyl, 1-indolyl, 2-indolyl, 3-indolyl, 4-indolyl, 5-indolyl, 6-indolyl, 7-indolyl, 1-isoindolyl, 2-isoindolyl, 3-isoindolyl, 4-isoindolyl, 5-isoindolyl, 6-isoindolyl, 7-isoindolyl, 2-furyl, 3-furyl, 2-benzofuranyl, 3-benzofuranyl, 4-benzofuranyl, 5-benzofuranyl, 6-benzofuranyl, 7-benzofuranyl, 1-isobenzofuranyl, 3-isobenzofuranyl, 4-isobenzofuranyl, 5-isobenzofuranyl, 6-isobenzofuranyl, 7-isobenzofuranyl, quinolyl, 3-quinolyl, 4-quinolyl, 5-quinolyl, 6-quinolyl, 7-quinolyl, 8-quinolyl, 1-isoquinolyl, 3-isoquinolyl, 4-isoquinolyl, 5-isoquinolyl, 6-isoquinolyl, 7-isoquinolyl, 8-isoquinolyl, 2-quinoxalinyl, 5-quinoxalinyl, 6-quinoxalinyl, 1-carbazolyl, 2-carbazolyl, 3-carbazolyl, 4-carbazolyl, 9-carbazolyl, 1-phenanthridinyl, 2-phenanthridinyl, 3-phenanthridinyl, 4-phenanthridinyl, 6-phenanthridinyl, 7-phenanthridinyl, 8-phenanthridinyl, 9-phenanthridinyl, 10-phenanthridinyl, 1-acridinyl, 2-acridinyl, 3-acridinyl, 4-acridinyl, 9-acridinyl, 1,7-phenanthroline-2-yl, 1,7-phenanthroline-3-yl, 1,7-phenanthroline-4-yl, 1,7-phenanthroline-5-yl, 1,7-phenanthroline-6-yl, 1,7-phenanthroline-8-yl, 1,7-phenanthroline-9-yl, 1,7-phenanthroline-10-yl, 1,8-phenanthroline-2-yl, 1,8-phenanthroline-3-yl, 1,8-phenanthroline-4-yl, 1,8-phenanthroline-5-yl, 1,8-phenanthroline-6-yl, 1,8-phenanthroline-7-yl, 1,8-phenanthroline-9-yl, 1,8-phenanthroline-10-yl, 1,9-phenanthroline-2-yl, 1,9-phenanthroline-3-yl, 1,9-phenanthroline-4-yl, 1,9-phenanthroline-5-yl, 1,9-phenanthroline-6-yl, 1,9-phenanthroline-7-yl, 1,9-phenanthroline-8-yl, 1,9-phenanthroline-10-yl, 1,10-phenanthroline-2-yl, 1,10-phenanthroline-3-yl, 1,10-phenanthroline-4-yl, 1,10-phenanthroline-5-yl, 2,9-phenanthroline-1-yl, 2,9-phenanthroline-3-yl, 2,9-phenanthroline-4-yl, 2,9-phenanthroline-5-yl, 2,9-phenanthroline-6-yl, 2,9-phenanthroline-7-yl, 2,9-phenanthroline-8-yl, 2,9-phenanthroline-10-yl, 2,8-phenanthroline-1-yl, 2,8-phenanthroline-3-yl, 2,8-phenanthroline-4-yl, 2,8-phenanthroline-5-yl, 2,8-phenanthroline-6-yl, 2,8-phenanthroline-7-yl, 2,8-phenanthroline-9-yl, 2,8-phenanthroline-10-yl, 2,7-phenanthroline-1-yl, 2,7-phenanthroline-3-yl, 2,7-phenanthroline-4-yl, 2,7-phenanthroline-5-yl, 2,7-phenanthroline-6-yl, 2,7-phenanthroline-8-yl, 2,7-phenanthroline-9-yl, 2,7-phenanthroline-10-yl, 1-phenazinyl, 2-phenazinyl, 1-phenothiazinyl, 2-phenothiazinyl, 3-phenothiazinyl, 4-phenothiazinyl, 10-phenothiazinyl, 1-phenoxazinyl, 2-phenoxazinyl, 3-phenoxazinyl, 4-phenoxazinyl, 10-phenoxazinyl, 2-oxazolyl, 4-oxazolyl, 5-oxazolyl, 2-oxadiazolyl, 5-oxadiazolyl, 3-furazanyl, 2-thienyl, 3-thienyl, 2-methylpyrrole-1-yl, 2-methylpyrrole-3-yl, 2-methylpyrrole-4-yl, 2-methylpyrrole-5-yl, 3-methylpyrrole-1-yl, 3-methylpyrrole-2-yl, 3-methylpyrrole-4-yl, 3-methylpyrrole-5-yl, 2-t-butylpyrrole-4-yl, 3-(2-phenylpropyl)pyrrole-1-yl, 2-methyl-1-indolyl, 4-methyl-1-indolyl, 2-methyl-3-indolyl, 4-methyl-3-indolyl, 2-t-butyl-1-indolyl, 4-t-butyl-1-indolyl, 2-t-butyl-3-indolyl and 4-t-butyl-3-indolyl.

Further, the heterocyclic group includes groups to which 1 to 10 benzene rings are bonded such as biphenyl, terphenyl and the like and groups having condensed rings such as naphthyl, anthranyl, phenanthryl, pyrenyl, coronyl and the like. Particularly preferred are groups to which 2 to 5 benzene rings are bonded and groups having a lot of meta bonds which bring about distortion to molecules.

The examples of the alkyl group represented by R described above include methyl, ethyl, propyl, isopropyl, n-butyl, s-butyl, isobutyl, t-butyl, n-pentyl, n-hexyl, n-heptyl, n-octyl, hydroxymethyl, 1-hydroxyethyl, 2-hydroxyethyl, 2-hydroxyisobutyl, 1,2-dihydroxyethyl, 1,3-dihydroxyisopropyl, 2,3-dihydroxy-t-butyl, 1,2,3-trihydroxypropyl, chloromethyl, 1-chloroethyl, 2-chloroethyl, 2-chloroisobutyl, 1,2-dichloroethyl, 1,3-dichloroisopropyl, 2,3-dichloro-t-butyl, 1,2,3-trichloropropyl, bromomethyl, 1-bromoethyl, 2-bromoethyl, 2-bromoisobutyl, 1,2-dibromoethyl, 1,3-dibromoisopropyl, 2,3-dibromo-t-butyl, 1,2,3-tribromopropyl, iodomethyl, 1-iodoethyl, 2-iodoethyl, 2-iodoisobutyl, 1,2-diiodoethyl, 1,3-diiodoisopropyl, 2,3-diiodo-t-butyl, 1,2,3-triiodopropyl, aminomethyl, 1-aminoethyl, 2-aminoethyl, 2-aminoisobutyl, 1,2-diaminoethyl, 1,3-diaminoisopropyl, 2,3-diamino-t-butyl, 1,2,3-triaminopropyl, cyanomethyl, 1-cyanoethyl, 2-cyanoethyl, 2-cyanoisobutyl, 1,2-dicyanoethyl, 1,3-dicyanoisopropyl, 2,3-dicyano-t-butyl, 1,2,3-tricyanopropyl, nitromethyl, 1-nitroethyl, 2-nitroethyl, 2-nitroisobutyl, 1,2-dinitroethyl, 1,3-dinitroisopropyl, 2,3-dinitro-t-butyl, 1,2,3-trinitropropyl, cyclopropyl, cyclobutyl, cyclopentyl, cyclohexyl, 4-methylcyclohexyl, 1-adamantyl, 2-adamantyl, 1-norbornyl, 2-norbornyl, cyclopropyl, cyclobutyl, cyclopentyl, cyclohexyl and 4-methylcyclohexyl.

The examples of the aralkyl group represented by R described above include benzyl, 1-phenylethyl, 2-phenylethyl, 1-phenylisopropyl, 2-phenylisopropyl, phenyl-t-butyl, α-naphthylmethyl, 1-α-naphthylethyl, 2-α-naphthylethyl, 1-α-naphthylisopropyl, 2-α-naphthylisopropyl, β-naphthylmethyl, 1-β-naphthylethyl, 2-β-naphthylethyl, 1-β-naphthylisopropyl, 2-β-naphthylisopropyl, 1-pyrrolylmethyl, 2-(1-pyrrolyl)ethyl, p-methylbenzyl, m-methylbenzyl, o-methylbenzyl, p-chlorobenzyl, m-chlorobenzyl, o-chlorobenzyl, p-bromobenzyl, m-bromobenzyl, o-bromobenzyl, p-iodobenzyl, m-iodobenzyl, o-iodobenzyl, p-hydroxybenzyl, m-hydroxybenzyl, o-hydroxybenzyl, p-aminobenzyl, m-aminobenzyl, o-aminobenzyl, p-nitrobenzyl, m-nitrobenzyl, o-nitrobenzyl, p-cyanobenzyl, m-cyanobenzyl, o-cyanobenzyl, 1-hydroxy-2-phenylisopropyl and 1-chloro-2-phenylisopropyl.

The alkoxy group in R described above is represented by - OY, and the examples of Y include the same ones as those of the alkyl group described above.
The aryloxy group in R described above is represented by -OY', and the examples of Y' include the same ones as those of the aryl group described above.
The arylthio group in R described above is represented by -SY', and the examples of Y' include the same ones as those of the aryl group described above.
The alkoxycarbonyl group in R described above is represented by -COOY, and the examples of Y include the same ones as those of the alkyl group described above.
Further, substituents for the respective groups described above include a halogen atom, a hydroxyl group, an amino group, a nitro group, a cyano group, an alkyl group, an alkenyl group, a cycloalkyl group, an alkoxy group, an aryl group, a heterocyclic group, an aralkyl group, an aryloxy group, an alkoxycarbonyl group, a carboxyl group and the like.

The condensed dicyclic group represented by Formula (1) described above is preferably a condensed dicyclic group represented by the following Formula (13), and the condensed dicyclic group represented by Formula (2) described above is preferably a condensed dicyclic group represented by the following Formula (14):

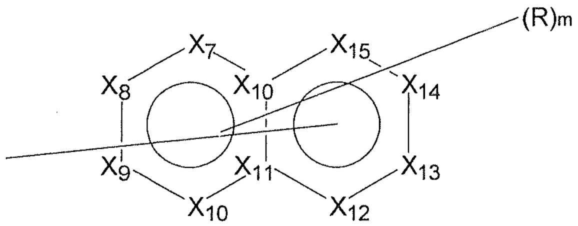

(13)

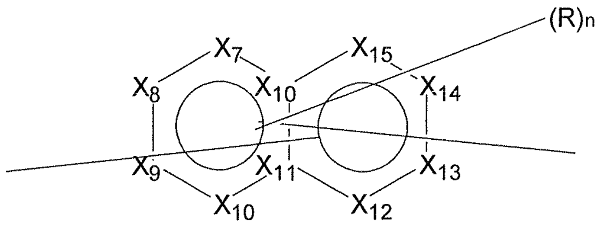

(14)

wherein $X_7$ to $X_{15}$ are the same as $X_1$ to $X_6$ described above, and R, m and n are the same as described above.

The examples of the condensed dicyclic group represented by Formula (1) or (2) described above include the residues of 1H-pyrrolidine, 1H-1-pyrindine, 1H-2-pyrindine, indolizine, 2H-isoindole, phthalamide, 1H-indole, skatole, indoxyl, indoline-3-one, isatin, 1H-indazole, indazoline, 7H-purine, xanthine, 2H-quinolizine, isoquinoline, isoquinolone, papaverine, quinoline, oxine, equinobuxin, quinaldic acid, 2,7-naphthyridine, 2,6-naphthyridine, phthalazine, 1,8-naphthyridine, 1,7-naphthyridine, 1,6-naphthyridine, 1,5-naphthyridine, quinoxaline, quinazoline, cinnoline, pteridine and the like. Among them, preferred are indazole, quinolizine and quinazoline residues (a quinazolinyl group or a quinazolinylene group) each having a nitrogen atom in a condensed part of two rings.
The above respective groups may have substituents, and the substituents are the same as in R described above.

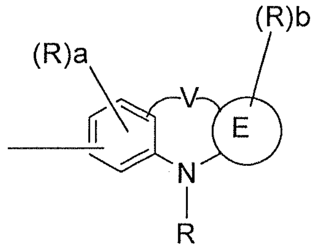
(3)

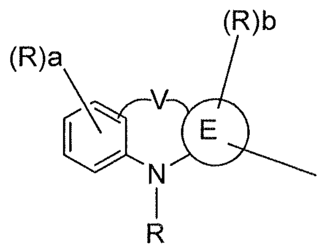
(4)

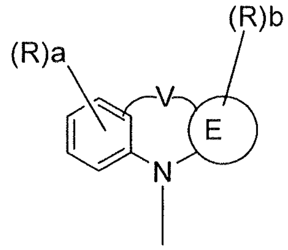
(5)

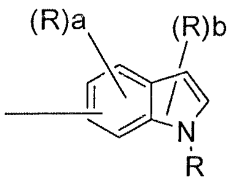
(6)

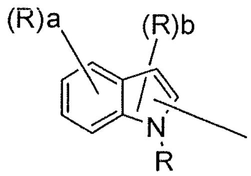
(7)

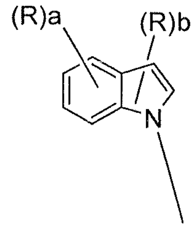
(8)

In Formulas (3) to (8), R is the same as described above, and a and b each are an integer of 0 to 4.

In Formulas (3) to (8), V is a single bond, $-CR_0R_0'-$, $-SiR_0R_0'-$, -O-, -CO- or $-NR_0$ ($R_0$ and $R_0'$ each are independently a hydrogen atom, an aryl group having 6 to 50 ring carbon atoms which may have a substituent, a heterocyclic group having 5 to 50 ring atoms which may have a substituent or an alkyl group having 1 to 50 carbon atoms which may have a substituent).
The examples of the aryl group, the heterocyclic group and the alkyl group represented by $R_0$ and $R_0'$ described above include the same ones as explained in R described above.

In Formulas (3) to (8), E represents a cyclic structure shown by a circle surrounding the symbol E and is a cycloalkane residue having 3 to 20 ring carbon atoms which may have a substituent and in which a carbon atom may be replaced by a nitrogen atom, an aromatic hydrocarbon group having 4 to 50 ring carbon atoms which may have a substituent or a heterocyclic group having 4 to 50 ring atoms which may have a substituent.
The examples of the cycloalkane residue represented by E described above include the residues of cyclopropane, cyclobutane, cyclohexane, cycloheptane, pyrrolidine, piperidine, piperazine and the like.
The aromatic hydrocarbon group represented by E described above includes the residues of benzene, naphthalene, anthracene, naphthacene, pyrene, chrysene, biphenyl, triphenylene, fluorene, bisfluorene and the like.
The heterocyclic group represented by E described above includes the residues of pyrazole, imidazole, pyrazine, pyrimidine, indazole, purine, phthalazine, naphthylidine, quinoxaline, quinazoline, cinnoline, pteridine, perimidine, phenanthroline, pyrroloimidazole, pyrrolotriazole, pyrazoloimidazole, pyrazolotriazole, pyrazolopyrimidine, pyrazolotriazine, imidazoimidazole, imidazopyridazine, imidazopyridine, imidazopyrazine, triazolopyridine, benzimidazole, naphthoimidazole, benzoxazole, naphthoxazole, benzothiazole, naphthothiazole, benzotriazole, tetrazaindene, triazine, carbazole and the like.

The carbazolyl group represented by Formula (3) described above includes structures represented by the following Formulas (15) to (18) (the carbazolyl group represented by Formula (4) includes the same structures):

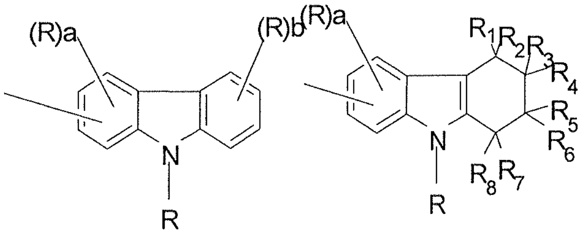

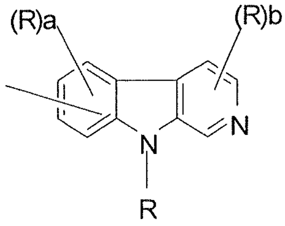

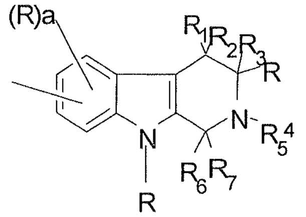

(15) (16) (17) (18)

(a, b and R are the same as described above, and $R_1$ to $R_8$ are the same as R).

Further, the specific examples of the structures represented by Formulas (15) to (18) include the following structures. Me represents methyl.

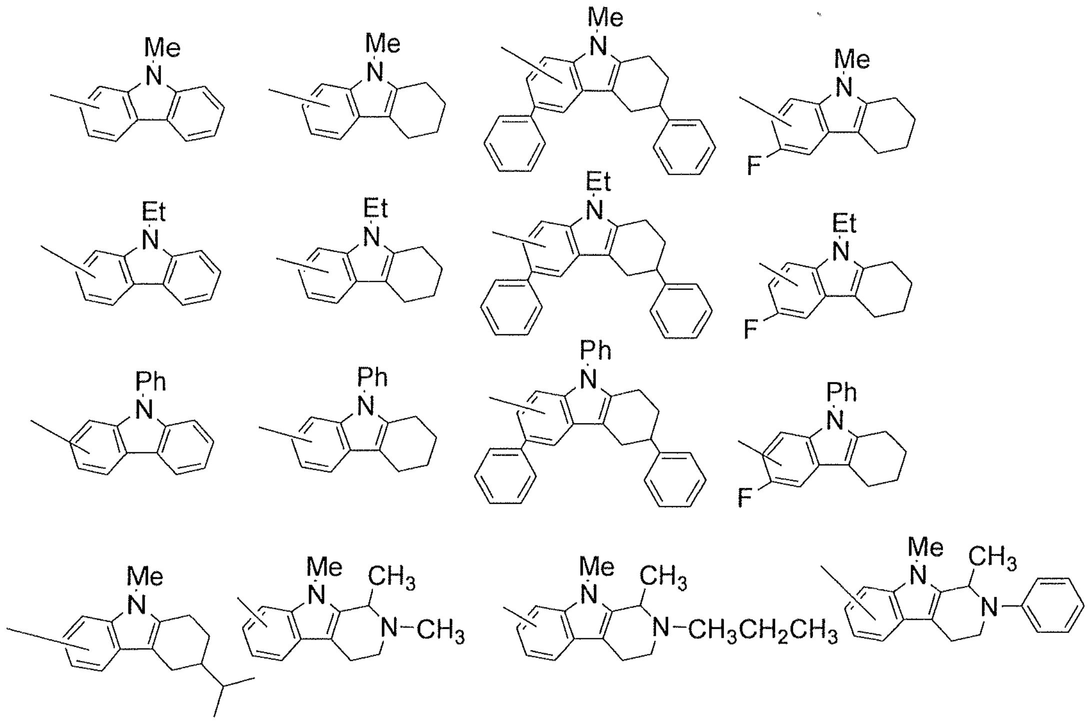

The carbazolyl group represented by Formula (5) described above includes structures represented by the following Formulas (19) to (22):

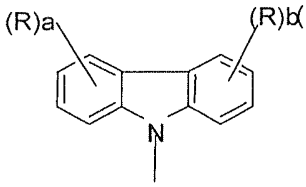

(19)

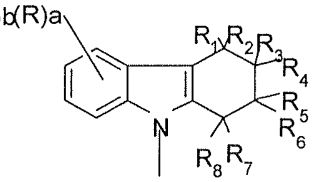

(20)

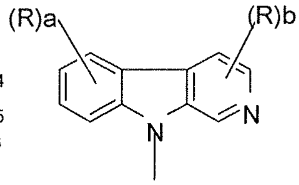

(21)

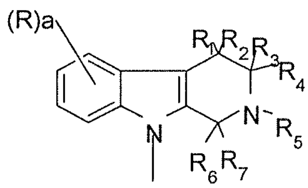

(22)

(a, b, R and $R_1$ to $R_8$ are the same as described above).

Further, the specific examples of the structures represented by Formulas (19) to (22) include the following structures:

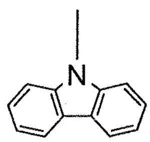
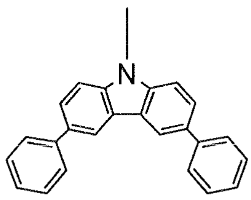
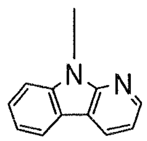
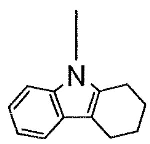
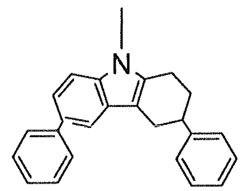
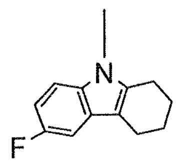

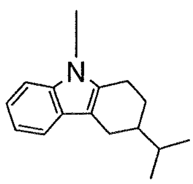
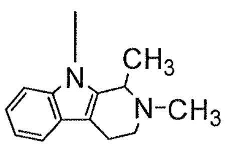
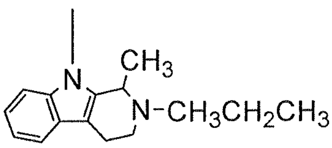
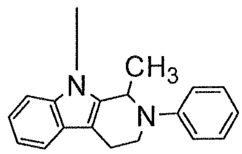

The specific examples of the indolyl group represented by Formula (6) described above includes the following structures (the indolyl group represented by Formula (7) includes the same structures):

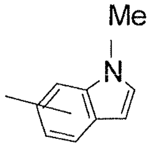
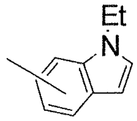
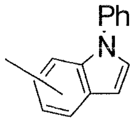
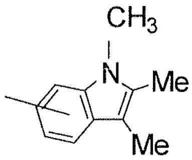
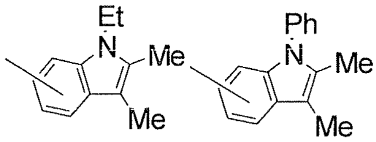

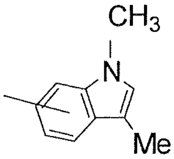
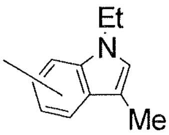
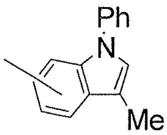
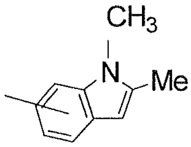
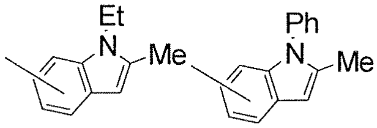

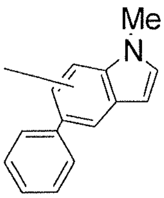
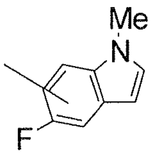

The specific examples of the indolyl group represented by Formula (8) described above includes the following structures:

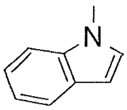
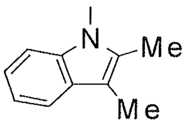
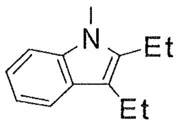
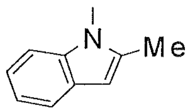
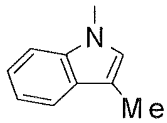

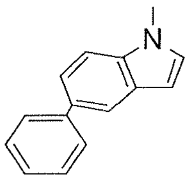
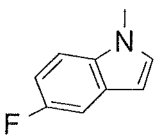

The condensed ring-containing compound of the present invention is preferably a condensed ring-containing compound represented by any of the following Formulas (9) to (12):

$$(C_Z-L)_{n1}-A \qquad (9)$$

$$(C_Z)_{n2}-L-A \qquad (10)$$

$$C_Z-L-(A)_{n3} \qquad (11)$$

$$L-(A-C_Z)_{n4} \qquad (12)$$

In Formulas (9) to (12), A is the condensed dicyclic group represented by Formula (1) and/or (2) described above, and when A is plural, they may be the same or different.
In Formulas (9) to (12), $C_z$ is the carbazolyl groups and/or the indolyl groups represented by any of Formulas (3) to (8) described above, and when $C_z$ is plural, they may be the same or different.
In Formulas (9) to (12), L is a single bond, an aromatic hydrocarbon group having 6 to 50 ring carbon atoms, a heterocyclic group having 2 to 50 ring carbon atoms, an aryl-substituted heterocyclic group having 2 to 50 ring carbon atoms, a diaryl-substituted heterocyclic group having 2 to 50 ring carbon atoms or a triaryl-substituted heterocyclic group having 2 to 50 ring carbon atoms. The above respective groups may have substituents, and when L is plural, they may be the same or different.
In Formula (9), n1 is an integer of 1 to 10, preferably an integer of 1 to 5; in Formula (10), n2 is an integer of 1 to 10, preferably an integer of 1 to 5; in Formula (11), n3 is an integer of 1 to 10, preferably an integer of 1 to 5; and in Formula (12), n4 is an integer of 1 to 10, preferably an integer of 1 to 5.
The aromatic hydrocarbon group represented by L described above includes, for example, the residues of benzene, naphthalene, anthracene, naphthacene, pyrene, chrysene, biphenyl, triphenylene, fluorene, bisfluorene and the like.
The heterocyclic group represented by L described above includes, for example, the residues of pyrazole, imidazole, pyrazine, pyrimidine, indazole, purine, phthalazine, naphthylidine, quinoxaline, quinazoline, cinnoline, pteridine, perimidine, phenanthroline, pyrroloimidazole, pyrrolotriazole, pyrazoloimidazole, pyrazolotriazole, pyrazolopyrimidine, pyrazolotriazine, imidazoimidazole, imidazopyridazine, imidazopyridine, imidazopyrazine, triazolopyridine, benzimidazole, naphthoimidazole, benzoxazole, naphthoxazole, benzothiazole, naphthothiazole, benzotriazole, tetrazaindene, triazine, carbazole and the like.
The aryl-substituted heterocyclic group, the diaryl-substituted heterocyclic group and the triaryl-substituted heterocyclic group represented by L described above include the heterocyclic groups described above which are substituted with the aromatic hydrocarbon groups described above.
The condensed ring-containing compound of the present invention is particularly preferably compounds having the following structures:

$C_z$-L-$Q_u$, $C_z$-L-$(Q_u)_2$, $C_z$-L-$Q_u$-L-$C_z$, $C_z$-L-$Q_u$-L'-$Q_u$, $C_z$-L-Het-$Q_u$, $C_z$-L-Het-$(Q_u)_2$, Cz-L-$Q_u$-Het

($C_z$ and L are the same as described above; L' is the same as L described above and different in a kind; $Q_u$ is a quinazolinyl group or a quinazolinylene group; and Het is a heterocyclic group.)
The specific examples of the condensed ring-containing compound of the present invention shall be shown below, but they shall not be restricted to these compounds shown as the examples.

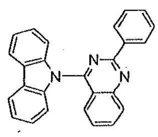
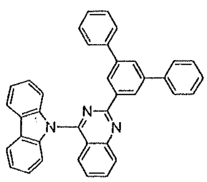
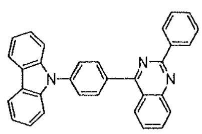
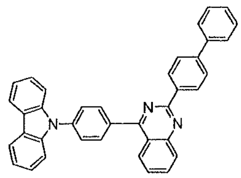

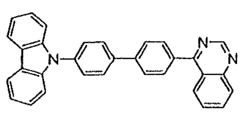
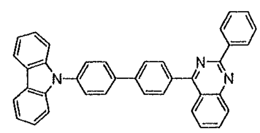
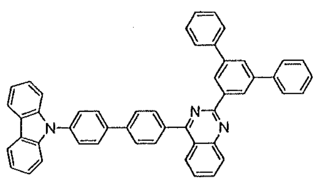
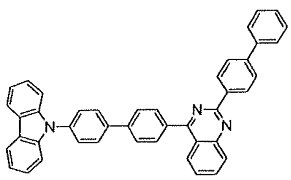

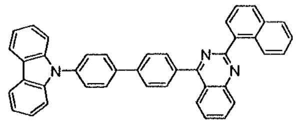 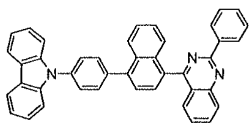 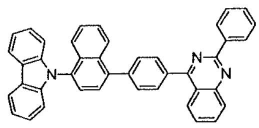 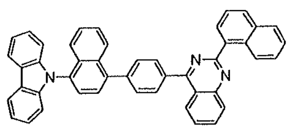

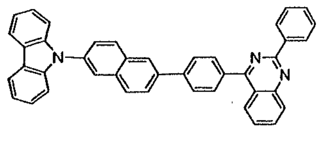 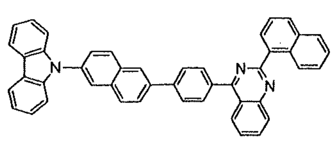

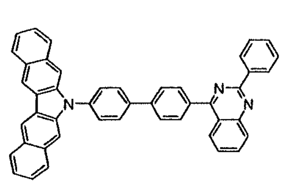 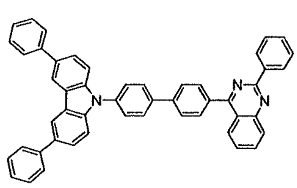 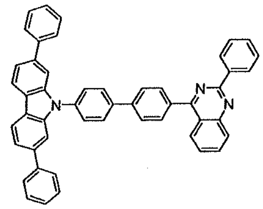 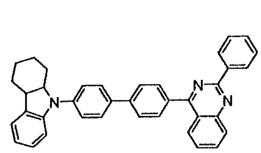

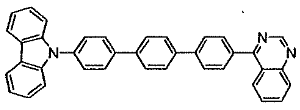 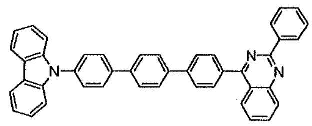 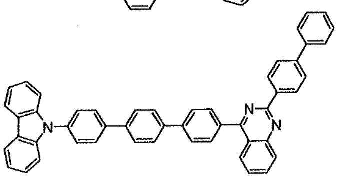

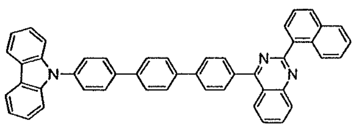 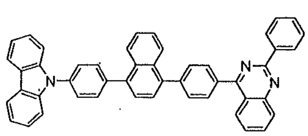 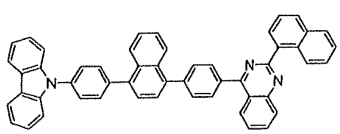

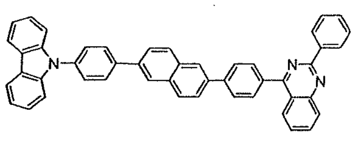 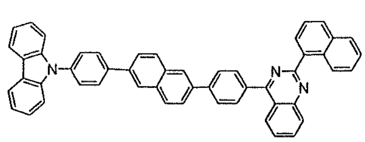

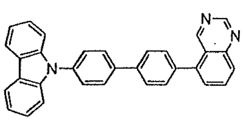 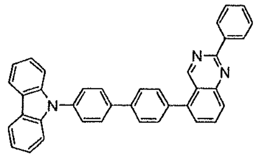 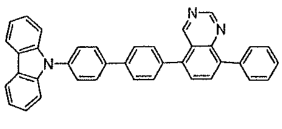 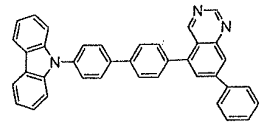

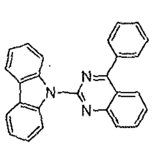 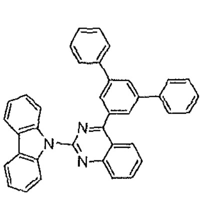 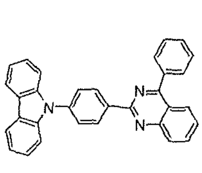 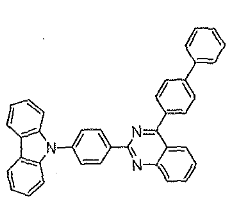

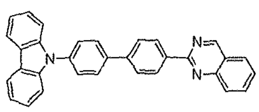
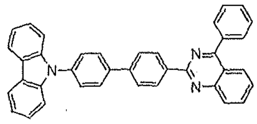
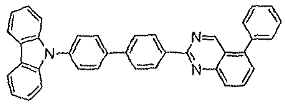
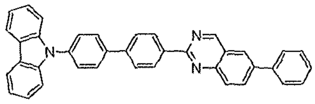
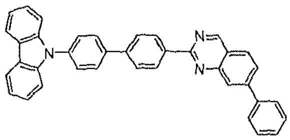
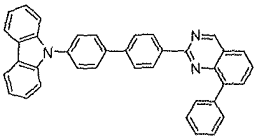
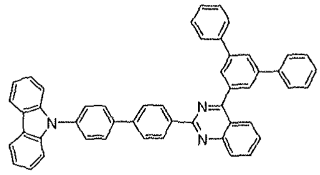
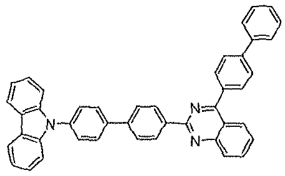
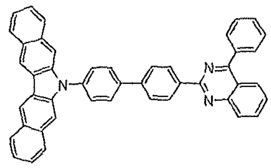
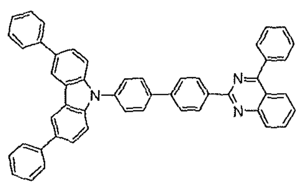
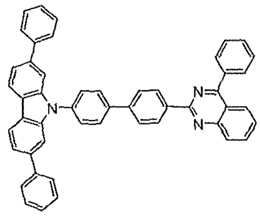
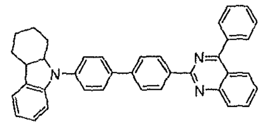
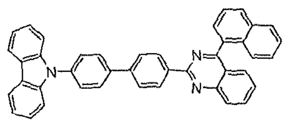
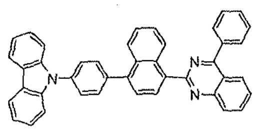
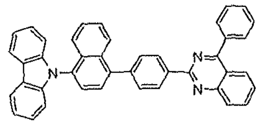
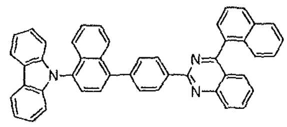
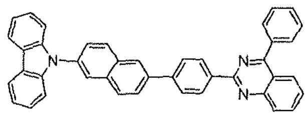
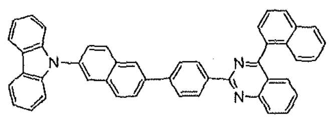
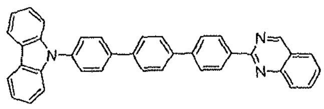
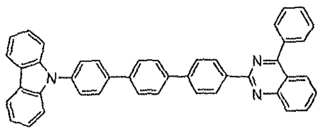
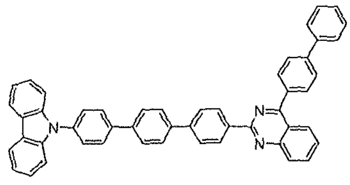
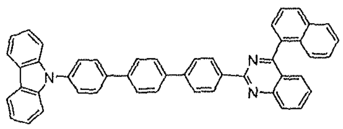
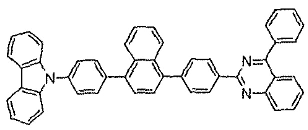
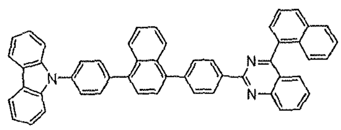
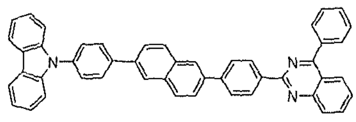
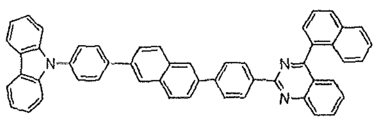

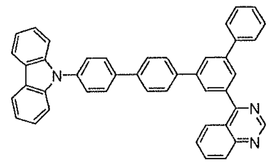
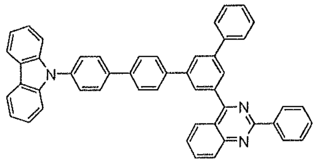
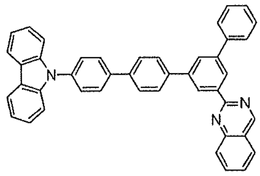
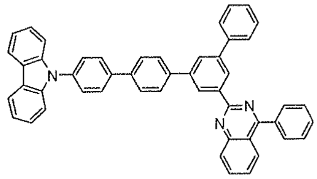

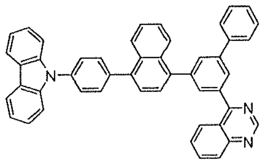
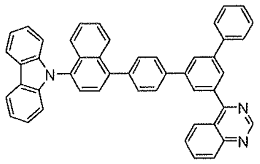
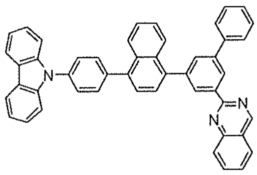
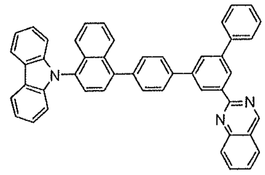

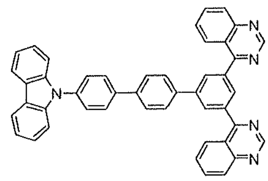
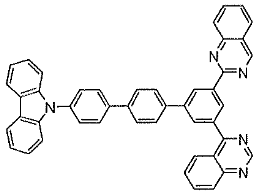
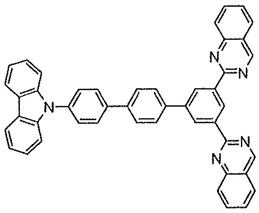
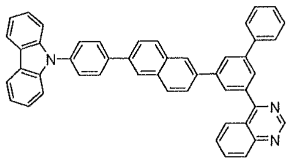

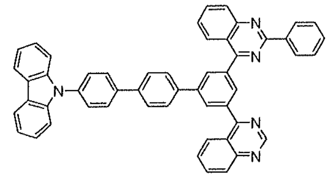
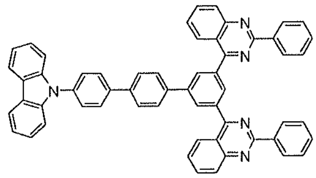
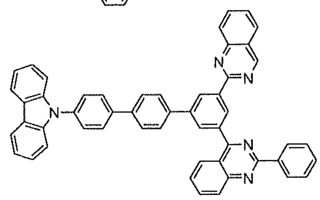

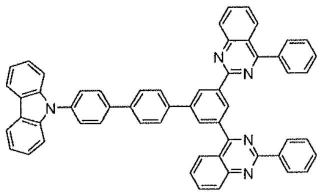
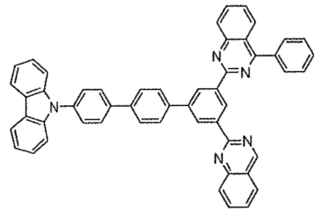
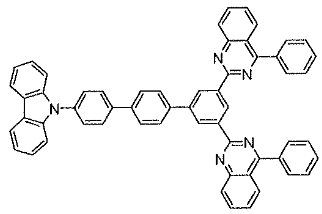

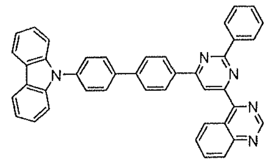
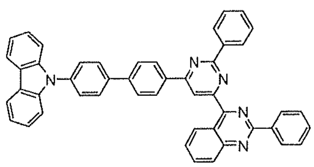
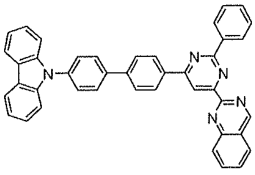
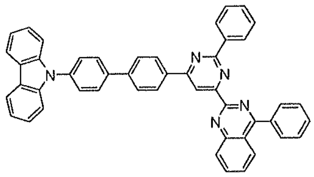

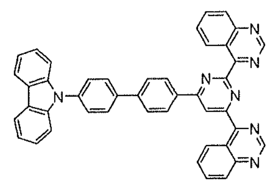
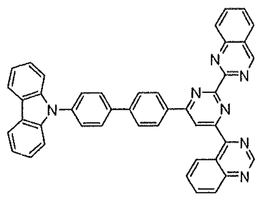
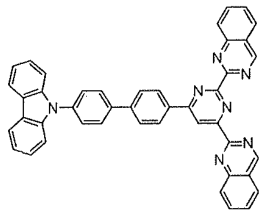
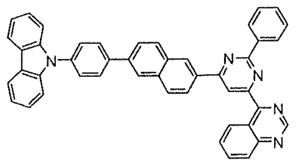

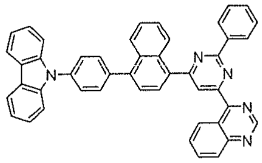

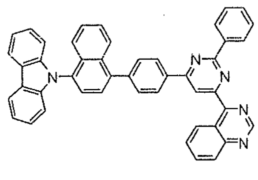

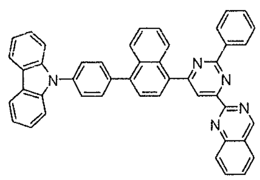

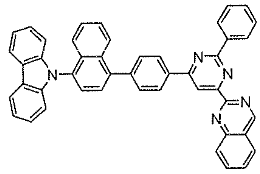

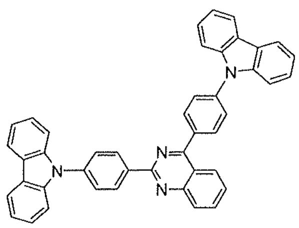

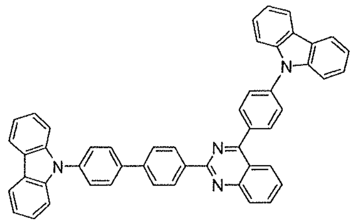

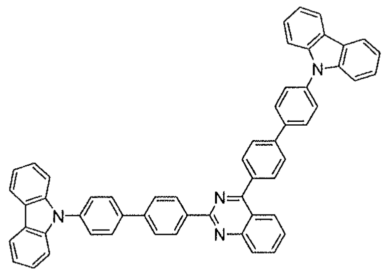

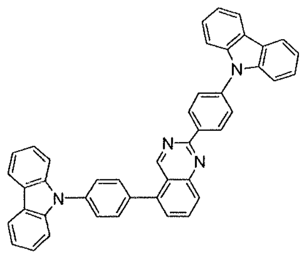

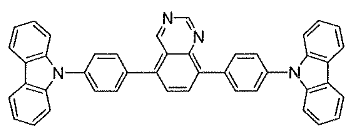

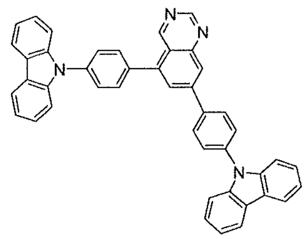

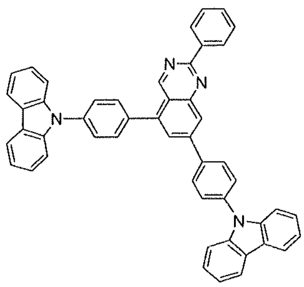

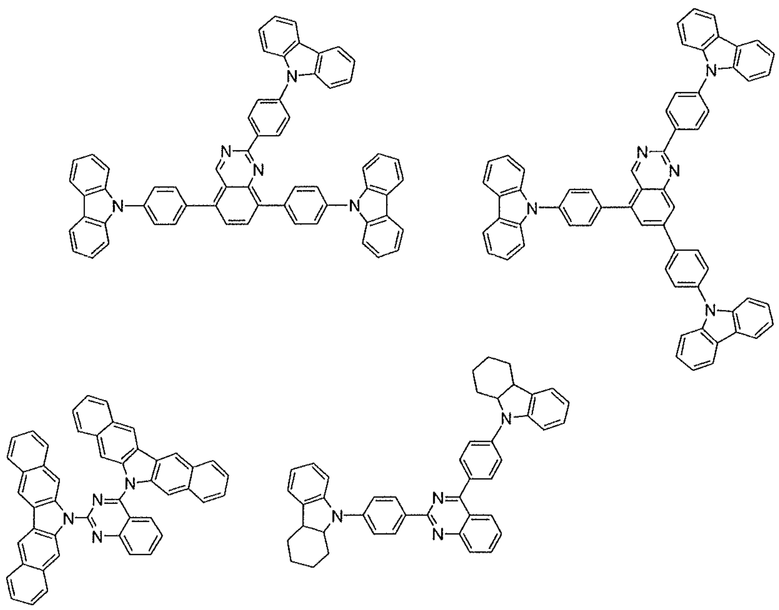

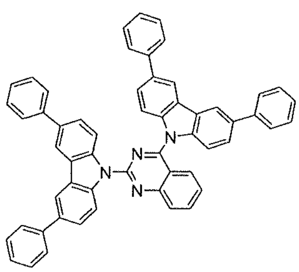

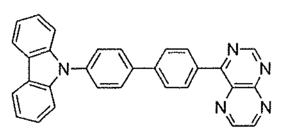

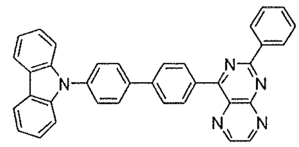

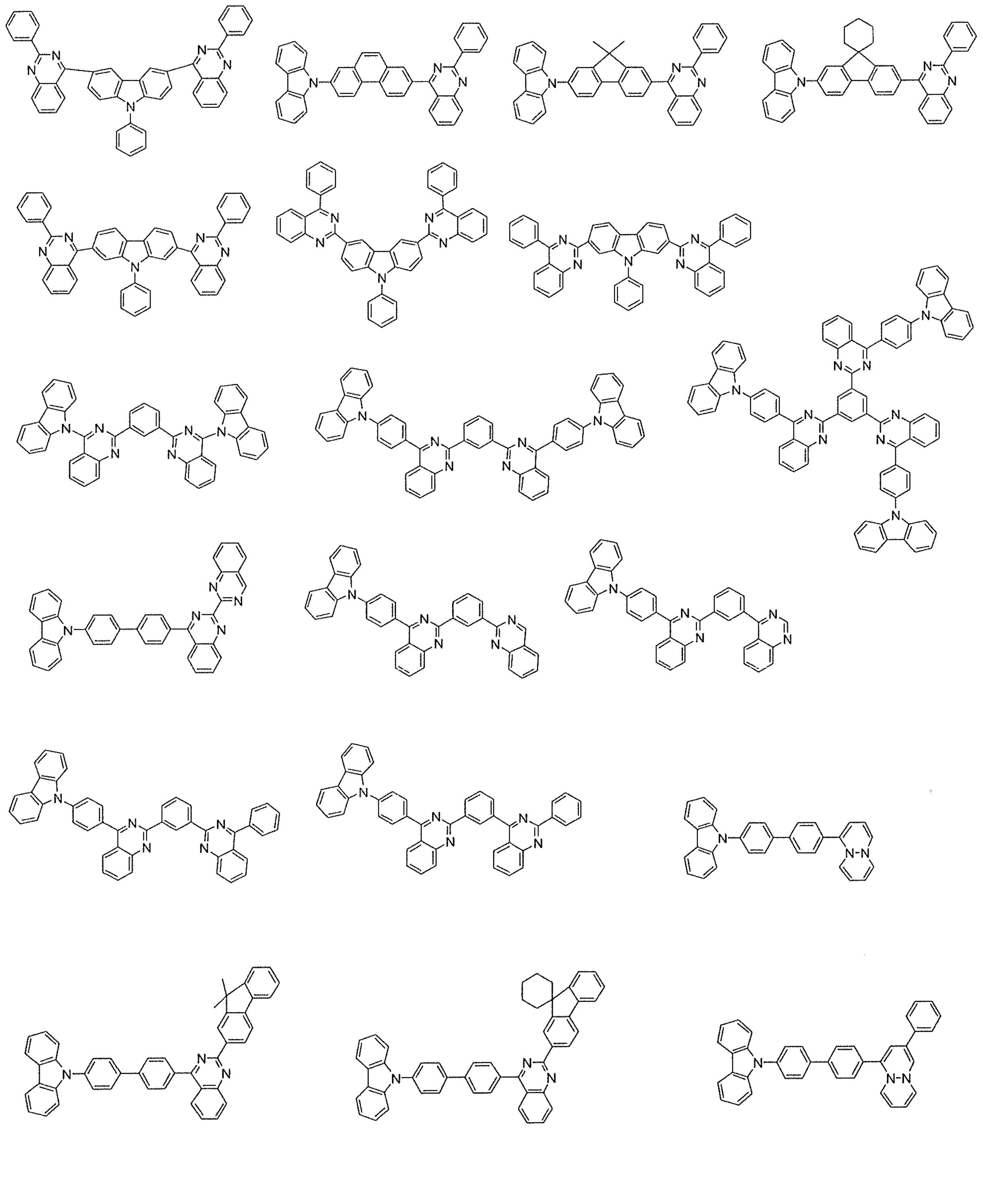

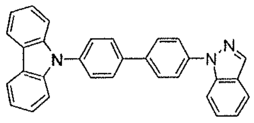
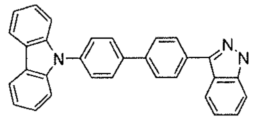
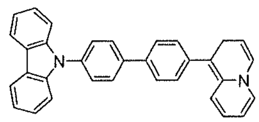
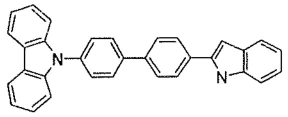
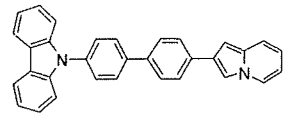
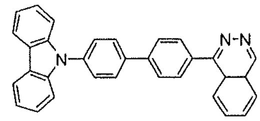

Next, the organic EL device of the present invention shall be explained.
The organic EL device of the present invention is an organic EL device in which an organic thin film layer comprising a single layer or plural layers having at least a light emitting layer is interposed between a cathode and an anode, wherein at least one layer in the above organic thin film layer contains the condensed ring-containing compound of the present invention in the form of a single component or a mixed component.
In the organic EL device of the present invention, the light emitting layer described above contains preferably the condensed ring-containing compound described above and a luminescent metal complex.
In the present invention, the condensed ring-containing compound described above is preferably a host material, and the luminescent metal complex described above is preferably a phosphorescent dopant. The reason therefor is that if the host material is the condensed ring-containing compound described above, combination thereof with the phosphorescent dopant described above makes it possible to make effective use of a triplet exciton state of the condensed ring-containing compound described above even on a room temperature condition (20°C). That is, a fluorescent luminous phenomenon can be brought about at a high current efficiency by allowing energy to effectively move from a triplet state brought about in the condensed ring-containing compound described above to the phosphorescent dopant.

In the condensed ring-containing compound of the present invention, a glass transition temperature is preferably 120°C or higher, and it falls in a range of more preferably 120 to 190°C, further preferably 130 to 180°C. If the glass transition temperature is 120°C or higher, the condensed ring-containing compound is less liable to be crystallized when combined with the phosphorescent dopant, and the lifetime is maintained for a long time. When applying current on a high temperature environmental condition, short circuit is less liable to be caused, and the use environment of the organic EL device is not limited. On the other hand, if the glass transition temperature is 190°C or lower, heat decomposition is less liable to be brought about when forming a film by vapor deposition, and handling thereof is easy. The glass transition temperature (Tg) can be determined in the form of a change point of a specific heat obtained when heating at a heating condition of, for example, 10° C/minute in a nitrogen circulating state by means of a differential scanning colorimeter (DSC).

In the organic EL device of the present invention, the relation of E1>E2 is preferably satisfied, wherein E1 is a triplet energy of the condensed ring-containing compound described above in the light emitting layer, and E2 is a value of a triplet energy of the phosphorescent dopant. That is, in the triplet energy relation described above, combination of the condensed ring-containing compound described above with the phosphorescent dopant makes it possible to surely make use of a triplet exciton state of the condensed ring-containing compound described above even on a room temperature condition. That is, a luminous phenomenon can be caused by allowing energy to surely move from a triplet excitation state brought about in the condensed ring-containing compound described above to the phosphorescent dopant.

The luminescent metal complex described above is, as described above, preferably a phosphorescent dopant, and it is preferably a metal complex containing at least one metal selected from the group consisting of Ir, Ru, Pd, Pt, Os and Re. The reason therefor is that if the phosphorescent dopant described above is the above metal complex, energy can effectively be transferred from the triplet exciton of the condensed ring-containing compound of the present invention.
The luminescent metal complex used in the present invention is preferably a metal complex having a structure represented by the following formula:

$$MY_p,\ MY_pY'_q,\ (MM')Y_p,\ (MM')Y_pY'_q,\ Y_p(MM')Y'_q$$

M, M': at least one metal selected from the group consisting of Ir, Ru, Pd, Pt, Os and Re and may have the same

kind of metal or different kinds of two or more metals in a molecule to become binuclear.

Y, Y': ligand, and the same kind of a ligand or different kinds of ligands may be coordinated according to a valence of metal.

p, q: an integer in which a valence of metal is an upper limit.

The luminescent metal complex used in the present invention shall not specifically be restricted as long as it can be used for an organic EL device, and a ligand of the metal complex has preferably at least one skeleton selected from the group consisting of a phenylpyridine skeleton, a phenylquinoline skeleton, a phenylisoquinoline skeleton, a bipyridyl skeleton, a phenanthroline skeleton and a benzothiophenepyridine skeleton. The reason therefor is that the above skeletons present in the molecule make it possible to effectively transfer energy from the triplet exciton of the compound having a condensed ring in a molecule.
The examples of the above luminescent metal complex include tris(2-phenylisoquinoline)iridium ($Ir(piq)_3$), tris(2-phenylpyridine)iridium, bis(2-phenylquinoline)iridium acetyl acetonate ($Ir(pq)_2(acac)$), bis(2-phenylisoquinoline)iridium acetyl acetonate ($Ir(piq)_2(acac)$), bis(2-benzothiophenepyridine)iridium acetyl acetonate ($Ir(btpy)_2(acac)$), tris(2-phenylpyridine)ruthenium, tris(2-phenylpyridine)palladium, bis(2-phenylpyridine)platinum, tris(2-phenylpyridine)osmium, tris(2-phenylpyridine)rhenium, octaethylplatinum porphyrin, octaphenylplatinum porphyrin, octaethylpalladium porphyrin and octaphenylpalladium porphyrin, and $Tr(piq)_3$, $Ir(pq)_2(acac)$, $Ir(piq)_2(acac)$ and $Ir(btpy)_2(acac)$b are preferred.
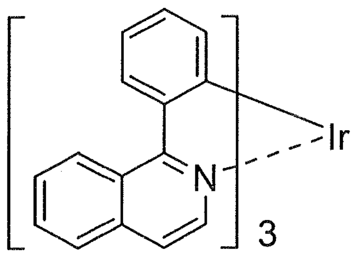

$Ir(piq)_3$

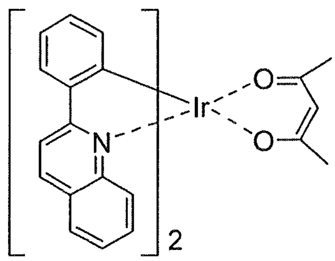

$Ir(pq)_2(acac)$

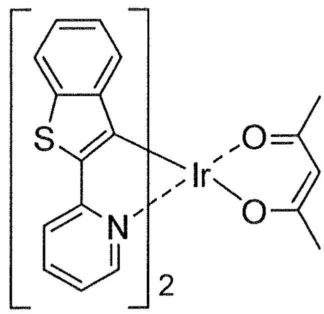

$Ir(btpy)_2(acac)$

In the present invention, a blending amount of the luminescent metal complex contained in the light emitting layer is preferably 0.1 to 50 parts by weight, more preferably 0.5 to 40 parts by weight and further preferably 1 to 30 parts by weight per 100 parts by weight of the condensed ring-containing compound (host material) described above. The reason therefor is that if a blending amount of the luminescent metal complex described above is 0.1 part by weight or more, an addition effect thereof is revealed to make it possible to effectively transfer energy from the triplet exciton of the condensed ring-containing compound described above and that if the blending amount is 50 parts by weight or less, it is easy to evenly blend the luminescent metal complex to prevent the emission luminance from scattering.
Structures which can be given as the typical device structure of the organic EL device of the present invention include:

(1) anode/light emitting layer/cathode,
(2) anode/hole injecting layer/light emitting layer/cathode,
(3) anode/light emitting layer/electron injecting layer/cathode,
(4) anode/hole injecting layer/light emitting layer/electron injecting layer/cathode,
(5) anode/organic semiconductor layer/luminescent layer/cathode,
(6) anode/organic semiconductor layer/electron barrier layer/light emitting layer/cathode,
(7) anode/organic semiconductor layer/light emitting layer/adhesion improving layer/cathode,
(8) anode/hole injecting layer/hole transporting layer/light emitting layer/electron injecting layer/cathode
(9) anode/insulating layer/light emitting layer/insulating layer/cathode,
(10) anode/inorganic semiconductor layer/insulating layer/light emitting layer/insulating layer/cathode
(11) anode/organic semiconductor layer/insulating, layer/light emitting layer/insulating layer/cathode,
(12) anode/insulating layer/hole injecting layer/hole transporting layer/light emitting layer/insulating layer/cathode and
(13) anode/insulating layer/hole injecting layer/hole transporting layer/light emitting layer/electron injecting layer/cathode

but it shall not be restricted to them.

The condensed ring-containing compound and the luminescent metal complex contained in the light emitting layer of the organic EL device of the present invention have been described previously.

Further, other publicly known luminescent materials (PVK, PPV, CBP, Alq, BAlq, publicly known complexes and the like) may be added, if necessary, to the light emitting layer as long as the object of the present invention is not damaged.

In the present invention, publicly known methods such as a vapor deposition method, a spin coating method, an LB method and the like can be applied as a method for forming the light emitting layer.

In the organic EL device of the present invention, a hole injecting layer having a thickness of 5 nm to 5 μm may be provided. Providing of such hole injecting layer improves injection of a hole into the light emitting layer and allows the high emission luminance to be obtained or makes driving at a low voltage possible. A compound having a hole mobility of $1\times 10^{-6}$ $cm^2/V\cdot$second or more measured when a voltage falling in a range of $1 \times 10^4$ to $1 \times 10^6$ V/cm is applied and an ionization energy of 5.5 eV or less is preferably used for the above hole injecting layer. A material for the above hole injecting layer includes, for example, porphyrin compounds, aromatic tertiary amine compounds, styrylamine compounds, aromatic dimethylidene base compounds and condensed aromatic ring compounds, and the specific examples thereof include organic compounds such as 4,4'-bis[N-(1-naphthyl)-N-phenylamino]biphenyl (abbreviated as NPD), 4,4',4"-tris[N-(3-methylphenyl)-N-phenylamino]triphenylamine (abbreviated as MTDATA) and the like. Further, two or more hole injecting layers are more preferably laminated if necessary. In this regard, when layers are laminated in the order of anode/hole injecting layer 1 (hole injecting material 1)/hole injecting layer 2 (hole injecting material 2)/ ···· /light emitting layer, an ionization energy (p) of the hole injecting material assumes preferably the form of Ip (hole injecting material 1)<Ip (hole injecting material 2) ... in terms of reducing the driving voltage.

Also, inorganic compounds such as p type-Si and p type-SiC are preferably used as a constitutional material for the hole injecting layer. Further, an organic semiconductor layer having an electric conductivity of $1 \times 10^{-10}$ s/cm or more is preferably provided as well between the hole injecting layer described above and an anode layer or between the hole injecting layer described above and the light emitting layer. Provision of the above organic semiconductor layer further improves injection of a hole into the light emitting layer.

In the organic EL device of the present invention, an electron injecting layer having a thickness of 5 nm to 5 μm may be provided. Provision of such electron injecting layer improves injection of an electron into the light emitting layer and allows the high emission luminance to be obtained or makes driving at a low voltage possible. A compound having an electron mobility of $1 \times 10^{-6}$ $cm^2/V\cdot$second or more measured when a voltage falling in a range of $1 \times 10^4$ to $1 \times 10^6$ V/cm is applied and an ionization energy of exceeding 5.5 eV is preferably used for the above electron e injecting layer. A material for the above electron injecting layer includes, for example, metal complexes (Al chelate: Alq) of 8-hydroxyquinoline or derivatives thereof, oxadiazole derivatives and the like.

Also, addition of alkali metal to the electron injecting layer makes it possible to markedly reduce the voltage and elongate the lifetime.

In the organic EL device of the present invention, a hole barrier layer having a thickness of 5 nm to 5 μm may be provided between the light emitting layer and the cathode. Provision of such hole barrier layer improves a confinement property of a hole into the organic light emitting layer and allows the high emission luminance to be obtained or makes driving at a low voltage possible. A material for the above hole barrier layer includes 2,9-dimethyl-4,7-diphenyl-1,10-phenanthroline, 2,9-diethyl-4,7-diphenyl-1,10-phenanthroline and the like, and alkali metals, for example, Li and Cs are preferably further added. As described above, combination of the material for the hole barrier layer with the alkali metal makes it possible to markedly reduce the voltage and elongate the lifetime. When adding the alkali metal, a content thereof is preferably 0.01 to 30 % by weight, more preferably 0.05 to 20 % by weight and further preferably 0.1 to 15 % by weight assuming that the whole amount of the hole barrier layer is 100 % by weight. The reason therefor is that if a content of the alkali metal is 0.01 % by weight or more, the addition effect is exhibited and that if the content is 30 % by weight or less, a dispersion of the alkali metal is uniform enough to prevent the emission luminance from scattering.

In the present invention, publicly known methods such as a vapor deposition method, a spin coating method, an LB method and the like can be applied as methods for forming the hole injecting layer, the electron injecting layer and the hole barrier layer each described above.

In the organic EL device of the present invention, a reducing dopant is preferably added to an interfacial region between the cathode and the organic thin film layer.

The reducing dopant includes at least one selected from alkali metals, alkali metal complexes, alkali metal compounds, alkaline earth metals, alkaline earth metal complexes, alkaline earth metal compounds, rare earth metals, rare earth metal complexes, rare earth metal compounds and halides and oxides thereof.

The alkali metal described above includes Li (work function: 2.93 eV),Na (work function: 2.36 eV), K (work function: 2.28 eV), Rb (work function: 2.16 eV), Cs (work function: 1.95 eV) and the like, and alkali metals having a work function of 3.0 eV or less are particularly preferred. Among them, Li, K, Rb and Cs are preferred.

The alkaline earth metal described above includes Ca (work function: 2.9 eV), Sr (work function: 2.0 to 2.5 eV), Ba (work function: 2.52 eV) and the like, and alkaline earth metals having a work function of 3.0 eV or less are particularly preferred.

The rare earth metal described above includes Sc, Y, Ce, Tb, Yb and the like, and rare earth metals having a work function of 3.0 eV or less are particularly preferred.
Among the metals described above, the preferred metals have particularly a high reducing ability and make it possible to enhance an emission luminance and elongate a lifetime in the organic EL device by adding a relatively small amount thereof to an electron injecting area.
The alkali metal compounds described above include alkali oxides such as $Li_2O$, $Cs_2O$, $K_2O$ and the like and alkali halides such as LiF, NaF, CsF, KF and the like, and alkali oxides or alkali halides such as LiF, $Li_2O$ and NaF are preferred.
The alkaline earth metal compounds described above include BaO, SrO, CaO and $Ba_xSr_{1-x}O$ ($0<x<1$) and $Ba_xCa_{1-x}$ ($0<x<1$) which are obtained by mixing the above compounds, and BaO, SrO and CaO are preferred.
The rare earth metal compounds described above include $YbF_3$, $ScF_3$, $ScO_3$, $Y_2O_3$, $Ce_2O_3$, $GdF_3$, $TbF_3$ and the like, and $YbF_3$, $ScF_3$ and $TbF_3$ are preferred.
The alkali metal complexes, the alkaline earth metal complexes and the rare earth metal complexes each described above shall not specifically be restricted as long as they contain at least one metal ion of alkali metal ions, alkaline earth metal ions and rare earth metal ions respectively. The ligands are preferably quinolinol, benzoquinolinol, acrydinol, phenanthridinol, hydroxyphenyloxazole, hydroxyphenylthiazole, hydroxydiaryloxadiazole, hydroxydiarylthiadiazole, hydroxyphenylpyridine, hydroxyphenylbenzimidazole, hydroxybenzotriazole, hydroxylfurborane, bipyridyl, phenanthroline, phthalocyanine, porphyrin, cyclopentadiene, β-diketones, azomethines and derivatives thereof. However the ligands shall not be restricted to the above compounds.
In an addition form of the reducing dopant, it is preferably formed in the shape of a layer or an island in the interfacial region described above. Preferred as a forming method therefor is a method in which a luminescent material for forming the interfacial region and an organic substance of an electron injecting material are deposited at the same time while depositing the reducing dopant by a resistance heating deposition method to thereby disperse the reducing dopant in the organic substance. The dispersion concentration is 100 : 1 to 1 : 100, preferably 5 : 1 to 1 : 5 in terms of a mole ratio of the organic substance to the reducing dopant.
When the reducing dopant is formed in the shape of a layer, the luminescent material and the electron injecting material which are used for the organic layers in the interface are formed in the shape of a layer, and then the reducing dopant is deposited alone by the resistance heating deposition method and formed preferably in a thickness of 0.1 to 15 nm.
When the reducing dopant is formed in the shape of an island, the luminescent material and the electron injecting material which are used for the organic layers in the interface are formed in the shape of an island, and then the reducing dopant is deposited alone by the resistance heating deposition method and preferably formed in a thickness of 0.05 to 1 nm.
In the organic EL device of the present invention, the anode corresponds to a lower electrode or a counter electrode according to the constitution of the organic EL display, and metal having a large work function (for example, 4.0 eV or more), alloy, an electrically conductive compound or a mixture thereof is preferably used for the above anode. To be specific, electrode materials such as indium tin oxide (ITO), indium zinc oxide (IZO), copper iodide (CuI), tin oxide ($Sn_2O$), zinc oxide (ZnO), gold, platinum, palladium and the like are preferably used alone or in combination of two or more kinds of the above electrode materials. Use of the above electrode materials makes it possible to form an anode having an uniform thickness by a method in which a film can be formed in a dry condition, such as a vacuum vapor deposition method, a sputtering method, an ion plating method, an electron beam vapor deposition method, a CVD (chemical vapor deposition) method, an MOCVD (metal oxide chemical vapor deposition) method, a plasma CVD method and the like. When taking out EL emission from the anode, the anode has to be a transparent electrode. In such case, a conductive transparent material such as ITO, IZO, CuI, $SnO_2$, ZnO and the like is preferably used to set a light transmittance of EL emission to a value of 70 % or more. A thickness of the anode shall not specifically be restricted, and it is a value falling in a range of preferably 10 to 1,000 nm, more preferably 10 to 200 nm. The reasons therefor are that the uniform thickness distribution and a light transmittance of 70 % or more in EL emission are obtained by setting a thickness of the anode to a value falling in the range described above and that a sheet resistance of the anode can be set to a value of 1,000 Ω/square or less, preferably 100 Ω/square or less. The anode (lower electrode), the organic luminescent medium and the cathode (counter electrode) are disposed in order, and preferably, the above lower electrode and counter electrode are constituted in the form of an XY matrix, whereby desired pixels on a luminous face are emitted. That is, constitution of the anode and the like in the manner described above makes it possible to readily display various information in the organic EL device.
In the organic EL device of the present invention, the cathode corresponds as well to a lower electrode or a counter electrode according to the constitution of the organic EL display, and metal having a small work function (for example, less than 4.0 eV), alloy, an electrically conductive compound or a mixture or an additive thereof is preferably used. To be specific, electrode materials comprising sodium, sodium-potassium alloys, cesium, magnesium, lithium, magnesium-silver alloys, aluminum, aluminum oxide, aluminum-lithium alloys, indium, rare earth metals, mixtures of the above metals with the materials for the organic thin film layer and mixtures of the above metals with the materials for the electron injecting layer are preferably used alone or in combination of two or more kinds of the above electrode

materials. A thickness of the cathode shall not specifically be restricted as is the case with the anode, and to be specific, it falls in a range of preferably 10 to 1,000 nm, more preferably 10 to 200 nm. When taking out EL emission from the cathode, the cathode has to be a transparent electrode. In such case, a light transmittance of EL emission is preferably set to a value of 70 % or more. The cathode is preferably formed, as is the case with the anode, by a method in which a film can be formed in a dry condition, such as a vacuum vapor deposition method, a sputtering method and the like.

A supporting substrate used in the organic EL device of the present invention is preferably excellent in a mechanical strength and has preferably a small permeability of moisture and oxygen, and the specific examples are glass plates, metal plates, ceramic plates and plastic plates (polycarbonate resins, acrylic resins, vinyl chloride resins, polyethylene terephthalate resins, polyimide resins, polyester resins, epoxy resins, phenol resins, silicone resins, fluororesins and the like). Further, the supporting substrate comprising the above materials is preferably provided with moisture-proof treatment and hydrophobic treatment by further forming thereon an inorganic film and coating with a fluororesin in order to prevent moisture from permeating the organic EL device. Also, a moisture content and a gas permeability coefficient in the supporting substrate are preferably reduced in order to prevent moisture from permeating particularly the organic thin film layer. To be specific, a moisture content of the supporting substrate is preferably set to 0.0001 % by weight or less, and a gas permeability coefficient thereof is preferably set to $1 \times 10^{-13}$ cc·cm/cm$^2$·sec·cm Hg or less.

EXAMPLES

Next, the present invention shall be explained in further details with reference to examples. The performances of organic EL devices obtained in the respective examples were evaluated in the following manners.

(1) Initial performance:

A prescribed voltage was applied to measure an electric current value at the moment, and the emission luminance value and the chromaticity coordinate at a CIEl932 chromaticity coordinate were measured at the same time by means of a color meter to evaluate the initial performance.

(2) Lifetime:

Constant current driving was carried out at an initial luminance of 1000 cd/m$^2$ to evaluate the lifetime by a half-lifetime of the luminance.

Example 1 (synthesis of compound (H-1) and production of organic EL device)

(1) Synthesis of compound (H-1)

A compound (H-1) was synthesized in the following manner.

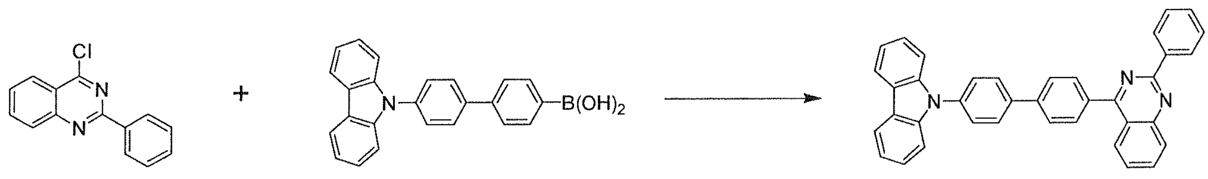

(H-1)

A 100 ml three neck flask was charged with 1.68 g (7 mmol) of 2-phenyl-4-chloroquinazoline (catalogue No. 16,243-4 manufactured by Aldrich Corp.), 2.80 g (7.7 mmol) of 4'-(N-carbazolyl)biphenyl borate and 0.243 g (0.21 mmol, 3 mol % Pd) of tetrakis-(triphenylphosphine)palladium (0), and the inside of the vessel was substituted with argon. Further, 26 ml of 1,2-dimethoxyethane and 12.5 ml (3 eq) of a 2M sodium carbonate aqueous solution were added thereto, and the mixture was heated and refluxed on an oil bath of 90°C for 9 hours. After finishing the reaction, resulting powder was taken by filtering to obtain 3.27 g of the compound (H-1). The compound (H-1) thus obtained was measured for FD-MS (field desorption mass spectrum), and the result thereof is shown below. FD-MS: calculated for $C_{38}H_{25}N_3$ = 524, found m/z=524 ($M^+$,100)
The compound (H-1) obtained was further refined by sublimation (340°C, $2 \times 10^{-3}$ Pa) and used for production of an organic EL device.

(2) Production of an organic EL device

A glass substrate of 25 mm × 75 mm × 0.7 mm thickness equipped with an ITO transparent electrode was subjected to supersonic wave washing in isopropyl alcohol for 5 minutes and then to UV ozone washing for 30 minutes. The glass substrate equipped with an ITO transparent electrode after washed was mounted on a substrate holder of a vacuum vapor depositing apparatus, and a copper phthalocyanine film (hereinafter abbreviated as "a CuPc film") having a film thickness of 10 nm was formed on a face at a side on which the transparent electrode was formed so that the transparent electrode described above was covered with the film. The above CuPc film functions as a hole injecting layer. The following 4,4'-bis[N-(1-naphthyl)-N-phenylamino]biphenyl film (hereinafter abbreviated as "an α-NPD film") having a film thickness of 30 nm was formed on the above CuPc film. The above α-NPD film functions as a hole transporting layer. Further, the compound (H-1) as a host material to which bis(2-phenylisoquinoline)iridium acetyl acetonate ($Ir(piq)_2(acac)$) of red color emission shown below was added as a phosphorescent Ir metal complex dopant was deposited to form a light emitting layer having a film thickness of 30 nm. A concentration of $Ir(piq)_2(acac)$ in the light emitting layer was set to 15 % by weight. A film of (1,1'-bisphenyl)-4-oleate)bis(2-methyl-quinolinolate)aluminum shown below (hereinafter abbreviated as "a BAlq film") having a film thickness of 10 nm was formed on the light emitting layer. The above BAlq film functions as a hole barrier layer. Further, a film of an aluminum complex of 8-hydroxyquinoline (hereinafter abbreviated as "an Alq film") having a film thickness of 40 nm was formed on the above film. The above Alq film functions as an electron injecting layer. Thereafter, LiF which is halogenated alkali metal was deposited thereon in a thickness of 0.2 nm, and then aluminum was deposited thereon in a thickness of 150 nm. This Al/LiF functions as a cathode. Thus, an organic EL device was produced.

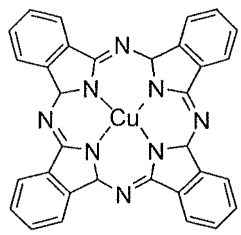

CuPc

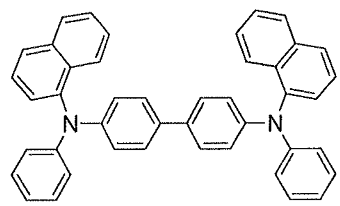

α −NPD

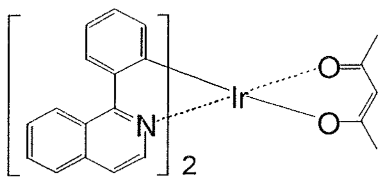

Ir(piq)2(acac)

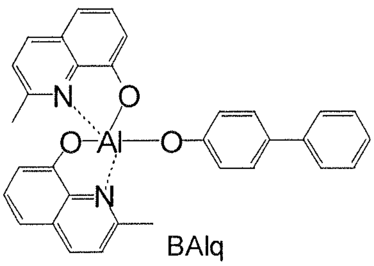

BAlq

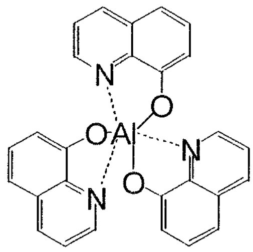

Alq

The device thus obtained was subjected to a current-carrying test to obtain red emission having an emission luminance of 101.0 cd/$m^2$ at a voltage of 5.5 V and a current density of 1.1 mA/$cm^2$, and a chromaticity coordinate of (0.668, 0.327) and current efficiency of 9.2 cd/A were obtained. Further, the above device was subjected to constant current driving at an initial luminance of 1000 cd/$m^2$ to find that the emission luminance was cut in half down to 500 cd/$m^2$ at a time of 2500 hours.

Example 2 (synthesis of compound (H-2) and production of organic EL device)

(1) Synthesis of compound (H-2)

A compound (H-2) was synthesized in the following manner.

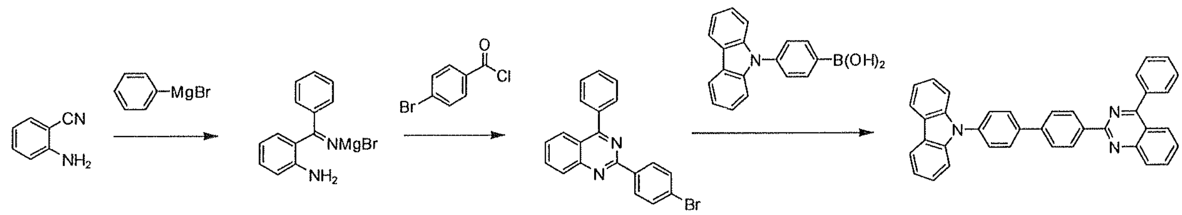

(Intermediate a) (Intermediate b) (H-2)

An intermediate b was synthesized by applying a method described in a document (J. Bergman, A. Brynolf, B. Elman and E. Vuorinen, Tetrahedron, 42, p. 3697 to 3706 (1986)). That is, a 500 ml three neck flask was charged with 100 ml (100 mmol) of a 1M tetrahydrofuran solution of phenylmagnesium bromide, and 100 ml of dried ether was added thereto, followed by heating and refluxing the mixture on an oil bath of 45°C. A dried ether 50 ml solution of 5.91 g (50 mmol) of 2-cyanoaniline was dropwise added thereto in 30 minutes. The solution was further refluxed for 1.5 hour and then cooled down to 0°C on an ice and water bath. Next, a dried ether 100 ml solution of 13.2 g (60 mmol) of 4-bromobenzoic chloride was dropwise added thereto in 10 minutes, and the solution was heated and refluxed on an oil bath of 45°C for 2 hours. After finishing the reaction, the solution was cooled down to 0°C on an ice and water bath, and a saturated ammonium chloride aqueous solution was added thereto. The deposit was filtered, washed with a small amount of methanol and then dried under vacuum to obtain 7.59 g of the intermediate b (yield: 42 %).
A 100 ml three neck flask was charged with 2.53 g (7 mmol) of the intermediate b, 2.07 g (7.7 mmol) of 4-(N-carbazolyl) phenyl borate and 0.243 g (0.21 mmol, 3 mol % Pd) of tetrakis (triphenylphosphine)-palladium (0), and the inside of the vessel was substituted with argon. Further, 26 ml of 1,2-dimethoxyethane and 12.5 ml (3 eq) of a 2M sodium carbonate aqueous solution were added thereto, and the mixture was heated and refluxed on an oil bath of 90°C for 9 hours. After finishing the reaction, resulting powder was taken by filtering to obtain 3.27 g of the compound (H-2). The compound (H-2) thus obtained was measured for FD-MS, and the result thereof is shown below.
FD-MS: calculated for $C_{38}H_{25}N_3$ = 524, found m/z=524 ($M^+$,100)
The compound (H-2) obtained was further refined by sublimation (340°C, 2 $\times$ $10^{-3}$ Pa) and used for production of an organic EL device.

(2) Production of an organic EL device

An organic EL device was produced in the same manner, except that in (2) of Example 1, the compound (H-2) obtained above was used as a host material for the light emitting layer in place of the compound (H-1).
The organic EL device thus obtained was evaluated for a current-carrying test and a life in the same manners as in Example 1, and the results thereof are shown in Table 1.

Example 3 (synthesis of compound (H-3) and production of organic EL device)

(1) Synthesis of compound (H-3)

A compound (H-3) was synthesized in the following manner.

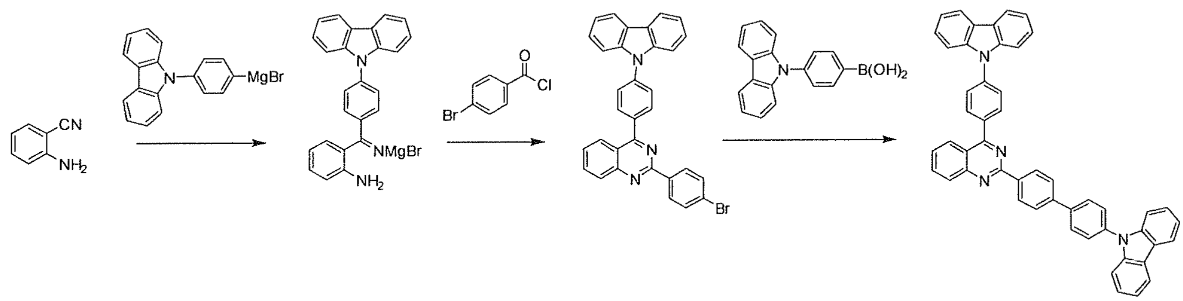

(Intermediate c) (Intermediate d) (H-3)

A 500 ml three neck flask was charged with 2.4 g (100 mmol) of magnesium and 100 ml of dried tetrahydrofuran, and 100 ml of a tetrahydrofuran solution of 35.4 g (110 mmol) of 4-(N-carbazolyl)phenyl bromide was added thereto to prepare a Grignard reagent. A dried tetrahydrofuran 50 ml solution of 5.91 g (50 mmol) of 2-cyanoaniline was dropwise added in 30 minutes to the above solution heated on an oil bath of 45°C. The solution was further heated for 1.5 hour to carry out reaction and then cooled down to 0°C on an ice and water bath. Next, a dried ether 100 ml solution of 13.2 g (60 mmol) of 4-bromobenzoic chloride was dropwise added thereto in 10 minutes, and the solution was heated on an oil bath of 45°C for 2 hours. After finishing the reaction, the solution was cooled down to 0°C on an ice and water bath, and a saturated ammonium chloride aqueous solution was added thereto. The deposit was filtered, washed with a small amount of methanol and then dried under vacuum to obtain 12.6 g of an intermediate d (yield: 48 %).
A 100 ml three neck flask was charged with 3.69 g (7 mmol) of the intermediate d, 2.07 g (7.7 mmol) of 4-(N-carbazolyl) phenyl borate and 0.243 g (0.21 mmol, 3 mol % Pd) of tetrakis(triphenylphosphine)-palladium (0), and the inside of the vessel was substituted with argon. Further, 26 ml of 1,2-dimethoxyethane and 12.5 ml (3 eq) of a 2M sodium carbonate aqueous solution were added thereto, and the mixture was heated and refluxed on an oil bath of 90°C for 9 hours. After finishing the reaction, resulting powder was taken by filtering to obtain 4.05 g of the compound (H-3). The compound (H-3) thus obtained was measured for FD-MS, and the result thereof is shown below.
FD-MS: calculated for $C_{50}H_{32}N_4$ = 689, found m/z=689 ($M^+$,100)
The compound (H-3) obtained was further refined by sublimation (390°C, $2 \times 10^{-3}$ Pa) and used for production of an organic EL device.

(2) Production of an organic EL device

An organic EL device was produced in the same manner, except that in (2) of Example 1, the compound (H-3) obtained above was used as a host material for the light emitting layer in place of the compound (H-1).
The organic EL device thus obtained was evaluated for a current-carrying test and a lifetime in the same manners as in Example 1, and the results thereof are shown in Table 1.

Example 4 (production of organic EL device)

An organic EL device was produced in the same manner, except that in (2) of Example 1, $Ir(pq)_2(acac)$ of orange emission described above was used as a dopant for the light emitting layer in place of $Ir(piq)_2(acac)$.
The organic EL device thus obtained was evaluated for a current-carrying test and a lifetime in the same manners as in Example 1, and the results thereof are shown in Table 1.

Comparative Example 1 (production of organic EL device)

An organic EL device was produced in the same manner, except that in Example 1, CBP shown below was used as a host material for the light emitting layer in place of the compound (H-1).
The organic EL device thus obtained was evaluated for a current-carrying test and a lifetime in the same manners as in Example 1, and the results thereof are shown in Table 1. As shown in Table 1, the organic EL device produced in Comparative Example 1 had a short lifetime and was impractical.

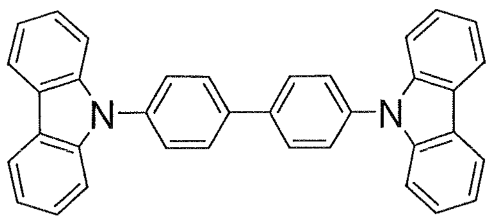

CBP

Table 1

| | Host material of light emitting layer | Dopant of light emitting layer | Voltage (V) | Current density ($mA/cm^2$) | Emission luminescence ($cd/m^2$) | Current efficiency (cd/A) | Chromaticity coordinate (x, y) | Half lifetime (hours) |
|---|---|---|---|---|---|---|---|---|
| Example 1 | H-1 | $Ir(piq)_2(acac)$ | 5.7 | 1.1 | 101.0 | 9.2 | (0.668, 0.327) | 25000 |
| Example 2 | H-2 | $Ir(piq)_2(acac)$ | 5.5 | 1.2 | 103.2 | 8.6 | (0.670, 0.325) | 23800 |
| Example 3 | H-3 | $Ir(piq)_2(acac)$ | 5.6 | 1.2 | 103.4 | 8.6 | (0.666, 0.329) | 22300 |
| Example 4 | H-1 | $Ir(pq)_2(acac)$ | 5.5 | 0.6 | 97.0 | 16.2 | (0.607, 0.386) | 23100 |
| Comparative Example 1 | CBP | $Ir(piq)_2(acac)$ | 5.9 | 1.4 | 103.3 | 7.4 | (0.662, 0.332) | 8300 |

As shown in Table 1, the light emitting layers were formed by using the condensed ring-containing compounds as a host material in the organic EL devices produced in Examples 1 to 4, whereby obtained was the notable effect that the lifetimes were double to triple as long as that of the organic EL device produced in Comparative Example 1 in which the publicly known compound CBP widely used as a phosphorescent material was used as a host material to form a light emitting layer.

INDUSTRIAL APPLICABILITY

As explained above in details, the organic EL devices using the condensed ring-containing compounds of the present invention have a high current efficiency and a high heat resistance and are elongated very much in a lifetime, and therefore they are practical.
Accordingly, the organic EL devices of the present invention are practical and useful as full color displays, information display equipments, car-mounted display equipments and lighting instruments.

**Claims**

1. A condensed ring-containing compound having a condensed dicyclic group represented by the following Formula (1) and/or (2) and carbazolyl groups and/or indolyl groups represented by at lest one selected from the following Formulas (3) to (8):

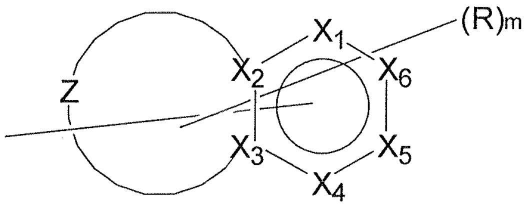

(1)

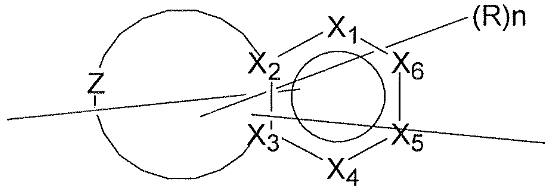

(2)

wherein $X_1$ to $X_6$ each are independently a nitrogen atom, an oxygen atom, a sulfur atom or a carbon atom; Z is an atomic group forming a cyclic structure; R is an aryl group having 6 to 50 ring carbon atoms which may have a substituent, a heterocyclic group having 5 to 50 ring atoms which may have a substituent, an alkyl group having 1 to 50 carbon atoms which may have a substituent, an alkoxy group having 1 to 50 carbon atoms which may have a substituent, an aralkyl group having 7 to 50 ring carbon atoms which may have a substituent, an aryloxy group having 5 to 50 ring carbon atoms which may have a substituent, an arylthio group having 5 to 50 ring carbon atoms which may have a substituent, an alkoxycarbonyl group having 1 to 50 carbon atoms which may have a substituent, a carboxyl group, a halogen atom, a cyano group, a nitro group or a hydroxyl group, and when R is plural, they may be combined with each other to form a cyclic structure; and
m and n each are an integer of 0 to 10;

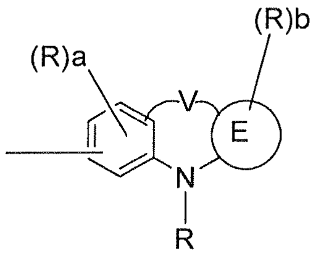 (3) 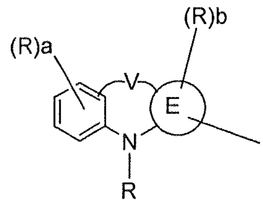 (4) 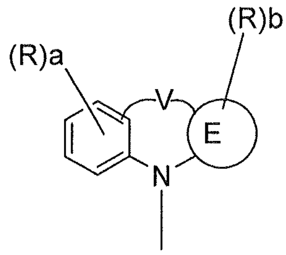 (5)

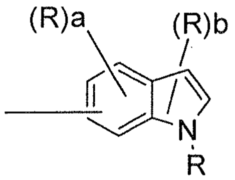 (6) 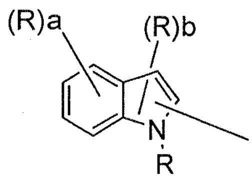 (7) 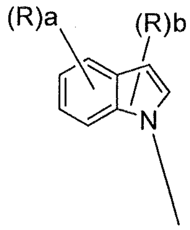 (8)

wherein R is the same as described above, and a and b each are an integer of 0 to 4;
V is a single bond, $CR_0R_0'$ -, $-SiR_0R_0'$-, -O-, -CO- or $-NR_0$ ($R_0$ and $R_0'$ each are independently a hydrogen atom, an aryl group having 6 to 50 ring carbon atoms which may have a substituent, a heterocyclic group having 5 to 50 ring atoms which may have a substituent or an alkyl group having 1 to 50 carbon atoms which may have a substituent);
E represents a cyclic structure shown by a circle surrounding the symbol E and is a cycloalkane residue having 3 to 20 ring carbon atoms which may have a substituent and in which a carbon atom may be replaced by a nitrogen atom, an aryl group having 4 to 50 ring carbon atoms which may have a substituent or a heterocyclic group having 4 to 50 ring atoms which may have a substituent.

**2.** The condensed ring-containing compound as described in claim 1, represented by any of the following Formulas (9) to (12) :

$$(C_Z-L)_{n1}-A \qquad (9)$$

$$(C_Z)_{n2}-L-A \qquad (10)$$

$$C_Z-L-(A)_{n3} \qquad (11)$$

$$L-(A-C_Z)_{n4} \qquad (12)$$

wherein A is the condensed dicyclic group represented by Formula (1) and/or (2) described above, and when A is plural, they may be the same or different;
$C_z$ is the carbazolyl groups and/or the indolyl groups represented by any of Formulas (3) to (8) described above, and when $C_z$ is plural, they may be the same or different; L is a single bond, an aromatic hydrocarbon group having 6 to 50 ring carbon atoms, a heterocyclic group having 2 to 50 ring carbon atoms, an aryl-substituted heterocyclic group having 2 to 50 ring carbon atoms, a diaryl-substituted heterocyclic group having 2 to 50 ring carbon atoms or a triaryl-substituted heterocyclic group having 2 to 50 ring carbon atoms; the above respective groups may have substituents, and when L is plural, they may be the same or different; n1 is an integer of 1 to 10; n2 is an integer of 1 to 10; n3 is an integer of 1 to 10; and n4 is an integer of 1 to 10.

**3.** The condensed ring-containing compound as described in claim 1 or 2, wherein the condensed dicyclic group

represented by Formula (1) described above is a condensed dicyclic group represented by the following Formula (13), and the condensed dicyclic group represented by Formula (2) described above is a condensed dicyclic group represented by the following Formula (14) :

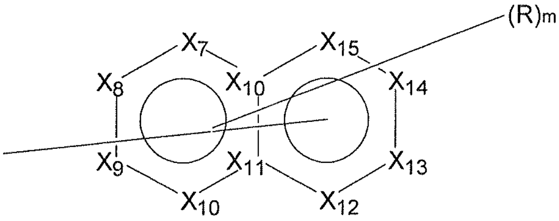

(13)

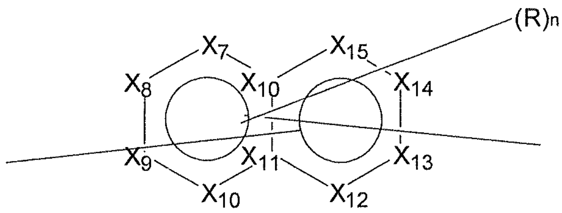

(14)

wherein $X_7$ to $X_{15}$ are the same as $X_1$ to $X_6$ described above, and R, m and n are the same as described above.

**4.** The condensed ring-containing compound as described in claim 1 or 2, wherein the condensed dicyclic group described above is a quinazolinyl group and/or a quinazolinylene group.

**5.** The condensed ring-containing compound as described in claim 1 or 2, wherein at least one of $X_1$, $X_2$ and $X_3$ is a nitrogen atom.

**6.** An organic electroluminescent device in which an organic thin film layer comprising a single layer or plural layers having at least a light emitting layer is interposed between a cathode and an anode, wherein at least one layer in the above organic thin film layer contains the condensed ring-containing compound as described in any of claims 1 to 5 in the form of a single component or a mixed component.

**7.** The organic electroluminescent device as described in claim 6, wherein the light emitting layer described above contains the condensed ring-containing compound described above and a luminescent metal complex.

**8.** The organic electroluminescent device as described in claim 6 or 7, containing the condensed ring-containing compound described above as a host material.

**9.** The organic electroluminescent device as described in any of claims 6 to 8, wherein a reducing dopant is added to an interfacial region between the cathode described above and the organic thin film layer described above.

**INTERNATIONAL SEARCH REPORT**

International application No. PCT/JP2005/019233

A. CLASSIFICATION OF SUBJECT MATTER
*C04D403/04*(2006.01), *C07D403/10*(2006.01), *C07D403/14*(2006.01), *C07D471/04*(2006.01), *C07D487/04*(2006.01), *H01L51/50*(2006.01)

According to International Patent Classification (IPC) or to both national classification and IPC

B. FIELDS SEARCHED

Minimum documentation searched (classification system followed by classification symbols)
*C04D403/04*(2006.01), *C07D403/10*(2006.01), *C07D403/14*(2006.01), *C07D471/04*(2006.01), *C07D487/04*(2006.01), *H01L51/50*(2006.01)

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
CAPLUS/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | JP 2004-256453 A (Dainippon Printing Co., Ltd.), 16 September, 2004 (16.09.04), Claim 1 (Family: none) | 1-3,5-9 |
| X | JP 2002-319491 A (Fuji Photo Film Co., Ltd.), 31 October, 2002 (31.10.02), Par. Nos. [0090] to [0108] & US 2002/055014 A1 & US 6824891 B2 | 1-3,5-9 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search: 17 November, 2005 (17.11.05) | Date of mailing of the international search report: 29 November, 2005 (29.11.05) |
| Name and mailing address of the ISA/ Japanese Patent Office | Authorized officer |
| Facsimile No. | Telephone No. |

Form PCT/ISA/210 (second sheet) (April 2005)

**INTERNATIONAL SEARCH REPORT**

International application No.
PCT/JP2005/019233

C (Continuation). DOCUMENTS CONSIDERED TO BE RELEVANT

| Category* | Citation of document, with indication, where appropriate, of the relevant passages | Relevant to claim No. |
|---|---|---|
| X | JENEKHE, S.A. et al., New Conjugated Polymers with Donor-Acceptor Architectures: Synthesis and Photophysics of Carbazole-Quinoline and Phenothiazine-Quinoline Copolymers and Oligomers Exhibiting Large Intramolecular Charge Transfer, Macromolecules, 2001, Vol.34, No.21, pages 7315 to 7324 | 1-3,5-9 |
| X | MEEKER, K. et al., Multicolor emission and tunale electroluminescence from blends of conjugated polymers, Polymeric Materials Science and Engineering, 2000, Vol.83, pages 208 to 209 | 1-3,5-9 |
| X | JP 03-203982 A (Mitsubishi Kasei Corp.), 05 September, 1991 (05.09.91), Page 6(XVI)<br>& EP 406762 A & JP 03-037293 A<br>& JP 03-037292 A & US 5059863 A<br>& EP 406762 B1 | 1-3,6-9 |
| X | US 2004/157084 A1 (SAMSUNG SDI CO., LTD.), 12 August, 2004 (12.08.04), Claim 2<br>& KR 2004072004 A & CN 1542083 A | 1,2,5-9 |
| X | WO 2003/080760 A1 (Idemitsu Kosan Co., Ltd.), 02 October, 2003 (02.10.03), pages 13 to 20<br>& US 2004/086745 A1 & EP 1489155 A1<br>& KR 2004094842 A & JP 2003-578493 A | 1,2,5-9 |
| X | JP 2001-284051 A (Fuji Photo Film Co., Ltd.), 12 October, 2001 (12.10.01), Par. No. [0017]<br>& US 2001/015614 A1 & JP 2001-172280 A<br>& JP 2001-278887 A & US 6461538 B2 | 1,2,5-9 |
| X | JP 2001-192653 A (Fuji Photo Film Co., Ltd.), 17 July, 2001 (17.07.01), Par. Nos. [0089] to [0108]<br>& US 6620529 B1 & US 2004/146745 A1 | 1,2,5-9 |
| X | US 2004/151943 A1 (LEE), 05 August, 2004 (05.08.04), Par. No. [0081]<br>(Family: none) | 1,2,6-9 |
| X | JP 2003-268362 A (Idemitsu Kosan Co., Ltd.), 25 September, 2003 (25.09.03), Par. Nos. [0009], [0010]<br>(Family: none) | 1,2,6-9 |

Form PCT/ISA/210 (continuation of second sheet) (April 2005)

**INTERNATIONAL SEARCH REPORT**

International application No.
PCT/JP2005/019233

C (Continuation). DOCUMENTS CONSIDERED TO BE RELEVANT

| Category* | Citation of document, with indication, where appropriate, of the relevant passages | Relevant to claim No. |
|---|---|---|
| X | MI, B. et al., Thermally Stable Hole-Transporting Material for Organic Light-Emitting Diode: an Isoindole Derivative, Chemistry of Materials, 2003, Vol.15, No.16, pages 3148 to 3151 | 1,2,6-9 |
| X | JP 56-120768 A (CIBA-Geigy AG.), 22 September, 1981 (22.09.81), Page 12, example 15 & EP 33716 A & BR 8100571 A & FI 8004067 A & EP 33716 B & US 4435003 A & CA 1162193 A & US 4480096 A & JP 89056103 B | 1-5 |
| P,X | JP 2005-289914 A (Canon Inc.), 20 October, 2005 (20.10.05), Par. Nos. [0042] to [0052] (Family: none) | 1-3,5-9 |
| P,X | JP 2004-363103 A (Canon Inc.), 24 December, 2004 (24.12.04), Par. No. [0030] & US 2004/247933 A1 | 1-3,5-9 |
| P,X | WO 2004/094389 A1 (Semiconductor Energy Laboratory Co., Ltd.), 04 November, 2004 (04.11.04), Pages 9 to 29 (Family: none) | 1-3,5-9 |
| P,X | WO 2005/076669 A1 (Idemitsu Kosan Co., Ltd.), 18 August, 2005 (18.08.05), Pages 18 to 27 (Family: none) | 1,2,5-9 |
| P,X | WO 2005/054212 A1 (CIBA SPECIALTY CHEMICALS HOLDING INC.), 16 June, 2005 (16.06.05), Page 29 (Family: none) | 1,2,6-9 |
| P,X | WO 2005/051046 A1 (CANON KABUSHIKI KAISHA), 02 June, 2005 (02.06.05), Pages 64, 66 & JP 2005-174917 A | 1,2,6-9 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2003031004 A
- JP 2003045662 A
- JP 2002193952 A
- JP 2002319419 A


### Non-patent literature cited in the description


- *Jpn. J. Appl. Phys.,* 1999, vol. 38, L1502
- **J. BERGMAN ; A. BRYNOLF ; B. ELMAN ; E. VUORINEN.** *Tetrahedron,* 1986, vol. 42, 3697-3706